# EUROPÄISCHE PATENTANMELDUNG

(11) **EP 3 404 107 A1**
(43) Veröffentlichungstag der Anmeldung: **21.11.2018**
(21) Anmeldenummer: 17172021.2
(22) Anmeldetag: 19.05.2017
(51) Int. Cl.: C12P 15/00, C12N 9/02, C12N 9/10, C12N 9/90

(54) **VERFAHREN ZUR FERMENTATIVEN DE NOVO SYNTHESE VON HARZSÄUREN**

(71) Anmelder: Jowat SE, 32758 Detmold (DE)
(72) Erfinder: Jach, Guido, 50678 Köln (DE); Schullehner, Katrin, 50678 Köln (DE); Welters, Peter, 41334 Nettetal (DE)
(74) Vertreter: dompatent von Kreisler Selting Werner - Partnerschaft von Patent- und Rechtsanwälten mbB

(57) **Zusammenfassung**

Die vorliegende Erfindung betrifft einen rekombinanten Mikroorganismus zur fermentativen *de novo* Synthese von Harzsäuregemischen definierter Zusammensetzung, wobei der rekombinante Mikroorganismus die Enzymaktivitäten wenigstens einer Geranylgeranyl-Pyrophosphat-Synthase (GGPPS), wenigstens einer Isopentenyl-Pyrophosphat-Isomerase (IPI), wenigstens einer Diterpensynthase (diTPS), wenigstens einer Cytochrom-P450-Monooxygenase (CYP), und wenigstens einer Cytochrom-P450-Reduktase (CPR) aufweist, wobei die Zusammensetzung im Wesentlichen durch die diTPS bestimmt wird.

Weiterhin betrifft die Erfindung ein Verfahren zur fermentativen *de novo* Synthese von Harzsäuregemischen definierter Zusammensetzung, umfassend die Kultivierung des rekombinanten Mikroorganismus.

Die vorliegende Erfindung betrifft auch die Verwendung des rekombinanten Mikroorganismus zur fermentativen *de novo* Synthese von Harzsäuregemischen definierter Zusammensetzung.

SEQUENCE LISTING

<110>  Jowat SE

<120>  Verfahren zur fermentativen de novo Synthese von Harzsäuren

<130>  161657ep

<160>  18

<170>  PatentIn version 3.3

<210>  1
<211>  2445
<212>  DNA
<213>  Ginkgo biloba

<400>  1
atgacagcgg cagagacacg gccggaccag ctgccgcaag aagaaaggtt cgtaagtcgc      60

ctaaatgccg attatcaccc tgccgtgtgg aaggacgact ttattgatag tttaaccagc     120

ccgaacagcc atgcaacctc caaatcgtct gttgatgaaa ctatcaataa acgtatccag     180

accttggtga aagaaataca atgcatgttt cagagcatgg gcgatggtga aacgaatcct     240

tctgcttacg ataccgcgtg ggtggcaaga ataccgtcga tcgacggcag tggcgcaccg     300

cagtttcccc agacacttca gtggatttta aacaatcagc tgccggatgg tagctggggt     360

gaagaatgca tctttttggc atatgatcga gtgctgaata cgttagcttg cttgcttacg     420

ctcaagatct ggaacaaagg cgatattcaa gttcagaaag gcgtcgagtt cgtacgtaaa     480

catatggagg agatgaaaga tgaggccgac aatcatcggc cttccgggtt cgaggtggta     540

tttccggcca tgttagatga agctaagagc cttggcctgg acctgccata ccatctgcct     600

tttattagcc agatccacca aaaacggcaa aagaaattac agaaaattcc gttaaatgtt     660

ttacataacc atcagacagc gcttttatac tctctcgagg ggctgcagga tgttgtcgat     720

tggcaggaaa ttactaatct tcaatcgcgt gatggaagtt ttctgagcag tccggcttcc     780

accgcttgtg tcttcatgca tacacaaaac aaacgctgtt tgcatttcct gaactttgtg     840

ttatctaagt tcggtgacta tgtaccttgt cactacccgt tggatctctt tgaacgcctg     900

tgggcggttg acacggtaga acgtctcggc atcgataggt attttaaaaa agaaattaaa     960

gaaagccttg attatgtgta ccgttactgg gacgcagaac gaggagtggg ctgggcgcga    1020

tgtaacccga ttccagatgt cgatgacacc gcaatggggc tgcgaatcct acgcctgcat    1080

ggctataacg tcagctccga cgtcctggag aatttccggg atgaaaaggg cgacttcttt    1140

tgcttcgcgg gccagactca gataggggtt accgataatc tgaatctgta ccgttgtagt    1200

caggtgtgct ttcctggtga aaaaattatg gaagaagcga agacgtttac gactaaccac    1260

ctgcaaaacg ccctggccaa aaataacgca ttcgataagt gggctgtcaa gaaggatctg    1320

ccgggcgaag tagaatacgc cattaagtat ccctggcatc gctccatgcc gcgtctggaa    1380

gcccgttcct atatcgagca gtttggttca aatgacgtct ggctcggtaa aacagtatac 1440

aaaatgctgt atgttagcaa cgagaaatat cttgagctgg ccaaactgga tttcaatatg 1500

gttcaagctt tgcaccaaaa ggaaacgcaa cacattgtgt catggtggcg cgagtctggc 1560

tttaatgatc tgacgtttac tcgtcagcgt ccggtagaaa tctatttctc cgtggcggtg 1620

agtatgttcg aaccagagtt cgccgcttgt cgcattgcct acgcaaaaac ctcatgcctc 1680

gcggtgatcc tggacgatct gtatgatacg catggtagtc tggatgacct caaattgttt 1740

agcgaggcgg tccgccgttg ggatattagt gttcttgaca gcgtgcgcga taaccaactg 1800

aaggtgtgtt ttctcggact gttcaacaca gtgaacggtt tcggcaaaga tgggctgaaa 1860

gaacagggcc gcgatgtctt gggttatttg cgcaaggtgt gggaaggttt gctagcgtcc 1920

tacacgaaag aggcggaatg gagcgcagca aagtatgtgc caacctttaa tgaatatgtc 1980

gagaatgcaa aagtttcaat tgcgctcgcc accgtagttc tgaattcaat cttctttacc 2040

ggcgagctgc tgccagatta cattctccag caggtagacc tgcgctcgaa attcttacat 2100

ctggtgagtc tcaccggccg tttgataaac gataccaaaa cataccaggc cgaacgtaac 2160

cgcggggagc tggtttcttc ggtccaatgt tacatgcgcg agaacccgga gtgtactgaa 2220

gaggaagctc tcagccacgt ctacggcata attgacaacg cactgaagga actgaactgg 2280

gagctagcaa accctgcgtc caacgcgccc ttatgcgtgc gccgtctgtt gtttaatacc 2340

gctcgtgtca tgcagctttt ttacatgtat cgtgacggtt ttggaatttc ggataaagaa 2400

atgaaagatc acgtgtcccg taccttattt gacccggtag catga 2445

<210> 2
<211> 814
<212> PRT
<213> Ginkgo biloba

<400> 2

Met Thr Ala Ala Glu Thr Arg Pro Asp Gln Leu Pro Gln Glu Glu Arg
1               5                   10                  15

Phe Val Ser Arg Leu Asn Ala Asp Tyr His Pro Ala Val Trp Lys Asp
            20                  25                  30

Asp Phe Ile Asp Ser Leu Thr Ser Pro Asn Ser His Ala Thr Ser Lys
        35                  40                  45

Ser Ser Val Asp Glu Thr Ile Asn Lys Arg Ile Gln Thr Leu Val Lys
    50                  55                  60

Glu Ile Gln Cys Met Phe Gln Ser Met Gly Asp Gly Glu Thr Asn Pro
65                  70                  75                  80

Ser Ala Tyr Asp Thr Ala Trp Val Ala Arg Ile Pro Ser Ile Asp Gly
                85                  90                  95

Ser Gly Ala Pro Gln Phe Pro Gln Thr Leu Gln Trp Ile Leu Asn Asn
            100                 105                 110

Gln Leu Pro Asp Gly Ser Trp Gly Glu Glu Cys Ile Phe Leu Ala Tyr
        115                 120                 125

Asp Arg Val Leu Asn Thr Leu Ala Cys Leu Leu Thr Leu Lys Ile Trp
    130                 135                 140

Asn Lys Gly Asp Ile Gln Val Gln Lys Gly Val Glu Phe Val Arg Lys
145                 150                 155                 160

His Met Glu Glu Met Lys Asp Glu Ala Asp Asn His Arg Pro Ser Gly
                165                 170                 175

Phe Glu Val Val Phe Pro Ala Met Leu Asp Glu Ala Lys Ser Leu Gly
            180                 185                 190

Leu Asp Leu Pro Tyr His Leu Pro Phe Ile Ser Gln Ile His Gln Lys
        195                 200                 205

Arg Gln Lys Lys Leu Gln Lys Ile Pro Leu Asn Val Leu His Asn His
    210                 215                 220

Gln Thr Ala Leu Leu Tyr Ser Leu Glu Gly Leu Gln Asp Val Val Asp
225                 230                 235                 240

Trp Gln Glu Ile Thr Asn Leu Gln Ser Arg Asp Gly Ser Phe Leu Ser
                245                 250                 255

Ser Pro Ala Ser Thr Ala Cys Val Phe Met His Thr Gln Asn Lys Arg
            260                 265                 270

Cys Leu His Phe Leu Asn Phe Val Leu Ser Lys Phe Gly Asp Tyr Val
        275                 280                 285

Pro Cys His Tyr Pro Leu Asp Leu Phe Glu Arg Leu Trp Ala Val Asp
    290                 295                 300

Thr Val Glu Arg Leu Gly Ile Asp Arg Tyr Phe Lys Lys Glu Ile Lys
305                 310                 315                 320

Glu Ser Leu Asp Tyr Val Tyr Arg Tyr Trp Asp Ala Glu Arg Gly Val

                325                 330                 335

Gly Trp Ala Arg Cys Asn Pro Ile Pro Asp Val Asp Asp Thr Ala Met
            340                 345                 350

Gly Leu Arg Ile Leu Arg Leu His Gly Tyr Asn Val Ser Ser Asp Val
        355                 360                 365

Leu Glu Asn Phe Arg Asp Glu Lys Gly Asp Phe Phe Cys Phe Ala Gly
    370                 375                 380

Gln Thr Gln Ile Gly Val Thr Asp Asn Leu Asn Leu Tyr Arg Cys Ser
385                 390                 395                 400

Gln Val Cys Phe Pro Gly Glu Lys Ile Met Glu Glu Ala Lys Thr Phe
                405                 410                 415

Thr Thr Asn His Leu Gln Asn Ala Leu Ala Lys Asn Asn Ala Phe Asp
            420                 425                 430

Lys Trp Ala Val Lys Lys Asp Leu Pro Gly Glu Val Glu Tyr Ala Ile
        435                 440                 445

Lys Tyr Pro Trp His Arg Ser Met Pro Arg Leu Glu Ala Arg Ser Tyr
    450                 455                 460

Ile Glu Gln Phe Gly Ser Asn Asp Val Trp Leu Gly Lys Thr Val Tyr
465                 470                 475                 480

Lys Met Leu Tyr Val Ser Asn Glu Lys Tyr Leu Glu Leu Ala Lys Leu
                485                 490                 495

Asp Phe Asn Met Val Gln Ala Leu His Gln Lys Glu Thr Gln His Ile
            500                 505                 510

Val Ser Trp Trp Arg Glu Ser Gly Phe Asn Asp Leu Thr Phe Thr Arg
        515                 520                 525

Gln Arg Pro Val Glu Ile Tyr Phe Ser Val Ala Val Ser Met Phe Glu
    530                 535                 540

Pro Glu Phe Ala Ala Cys Arg Ile Ala Tyr Ala Lys Thr Ser Cys Leu
545                 550                 555                 560

Ala Val Ile Leu Asp Asp Leu Tyr Asp Thr His Gly Ser Leu Asp Asp
                565                 570                 575

Leu Lys Leu Phe Ser Glu Ala Val Arg Arg Trp Asp Ile Ser Val Leu
            580                 585                 590

Asp Ser Val Arg Asp Asn Gln Leu Lys Val Cys Phe Leu Gly Leu Phe
        595                 600                 605

Asn Thr Val Asn Gly Phe Gly Lys Asp Gly Leu Lys Glu Gln Gly Arg
    610                 615                 620

Asp Val Leu Gly Tyr Leu Arg Lys Val Trp Glu Gly Leu Leu Ala Ser
625                 630                 635                 640

Tyr Thr Lys Glu Ala Glu Trp Ser Ala Ala Lys Tyr Val Pro Thr Phe
                645                 650                 655

Asn Glu Tyr Val Glu Asn Ala Lys Val Ser Ile Ala Leu Ala Thr Val
            660                 665                 670

Val Leu Asn Ser Ile Phe Phe Thr Gly Glu Leu Leu Pro Asp Tyr Ile
        675                 680                 685

Leu Gln Gln Val Asp Leu Arg Ser Lys Phe Leu His Leu Val Ser Leu
    690                 695                 700

Thr Gly Arg Leu Ile Asn Asp Thr Lys Thr Tyr Gln Ala Glu Arg Asn
705                 710                 715                 720

Arg Gly Glu Leu Val Ser Ser Val Gln Cys Tyr Met Arg Glu Asn Pro
                725                 730                 735

Glu Cys Thr Glu Glu Glu Ala Leu Ser His Val Tyr Gly Ile Ile Asp
            740                 745                 750

Asn Ala Leu Lys Glu Leu Asn Trp Glu Leu Ala Asn Pro Ala Ser Asn
        755                 760                 765

Ala Pro Leu Cys Val Arg Arg Leu Leu Phe Asn Thr Ala Arg Val Met
    770                 775                 780

Gln Leu Phe Tyr Met Tyr Arg Asp Gly Phe Gly Ile Ser Asp Lys Glu
785                 790                 795                 800

Met Lys Asp His Val Ser Arg Thr Leu Phe Asp Pro Val Ala
                805                 810

<210>  3
<211>  939
<212>  DNA

<213>  Pantoea agglomerans

<400>  3
atgcattatc cgtttataag gacagcccga atgacggtct gcgcaaaaaa acacgttcat      60

ctcactcgcg atgctgcgga gcagttactg gctgatattg atcgacgcct tgatcagtta     120

ttgcccgtgg agggagaacg ggatgttgtg ggtgccgcga tgcgtgaagg tgcgctggca     180

ccgggaaaac gtattcgccc catgttgctg ttgctgaccg cccgcgatct gggttgcgct     240

gtcagccatg acggattact ggatttggcc tgtgcggtgg aaatggtcca cgcggcttcg     300

ctgatccttg acgatatgcc ctgcatggac gatgcgaagc tgcggcgcgg acgccctacc     360

attcattctc attacggaga gcatgtggca atactggcgg cggttgcctt gctgagtaaa     420

gcctttggcg taattgccga tgcagatggc ctcacgccgc tggcaaaaaa tcgggcggtt     480

tctgaactgt caaacgccat cggcatgcaa ggattggttc agggtcagtt caaggatctg     540

tctgaagggg ataagccgcg cagcgctgaa gctattttga tgacgaatca ctttaaaacc     600

agcacgctgt tttgtgcctc catgcagatg gcctcgattg ttgcgaatgc ctccagcgaa     660

gcgcgtgatt gcctgcatcg tttttcactt gatcttggtc aggcatttca actgctggac     720

gatttgaccg atggcatgac cgacaccggt aaggatagca atcaggacgc cggtaaatcg     780

acgctggtca atctgttagg cccgagggcg gttgaagaac gtctgagaca acatcttcag     840

cttgccagtg agcatctctc tgcggcctgc caacacgggc acgccactca acattttatt     900

caggcctggt ttgacaaaaa actcgctgcc gtcagttaa                            939


<210>  4
<211>  312
<212>  PRT
<213>  Pantoea agglomerans

<400>  4

Met His Tyr Pro Phe Ile Arg Thr Ala Arg Met Thr Val Cys Ala Lys
1               5                   10                  15


Lys His Val His Leu Thr Arg Asp Ala Ala Glu Gln Leu Leu Ala Asp
            20                  25                  30


Ile Asp Arg Arg Leu Asp Gln Leu Leu Pro Val Glu Gly Glu Arg Asp
        35                  40                  45


Val Val Gly Ala Ala Met Arg Glu Gly Ala Leu Ala Pro Gly Lys Arg
    50                  55                  60


Ile Arg Pro Met Leu Leu Leu Leu Thr Ala Arg Asp Leu Gly Cys Ala
65                  70                  75                  80

Val Ser His Asp Gly Leu Leu Asp Leu Ala Cys Ala Val Glu Met Val
                85                  90                  95

His Ala Ala Ser Leu Ile Leu Asp Asp Met Pro Cys Met Asp Asp Ala
            100                 105                 110

Lys Leu Arg Arg Gly Arg Pro Thr Ile His Ser His Tyr Gly Glu His
        115                 120                 125

Val Ala Ile Leu Ala Ala Val Ala Leu Leu Ser Lys Ala Phe Gly Val
    130                 135                 140

Ile Ala Asp Ala Asp Gly Leu Thr Pro Leu Ala Lys Asn Arg Ala Val
145                 150                 155                 160

Ser Glu Leu Ser Asn Ala Ile Gly Met Gln Gly Leu Val Gln Gly Gln
                165                 170                 175

Phe Lys Asp Leu Ser Glu Gly Asp Lys Pro Arg Ser Ala Glu Ala Ile
            180                 185                 190

Leu Met Thr Asn His Phe Lys Thr Ser Thr Leu Phe Cys Ala Ser Met
        195                 200                 205

Gln Met Ala Ser Ile Val Ala Asn Ala Ser Ser Glu Ala Arg Asp Cys
    210                 215                 220

Leu His Arg Phe Ser Leu Asp Leu Gly Gln Ala Phe Gln Leu Leu Asp
225                 230                 235                 240

Asp Leu Thr Asp Gly Met Thr Asp Thr Gly Lys Asp Ser Asn Gln Asp
                245                 250                 255

Ala Gly Lys Ser Thr Leu Val Asn Leu Leu Gly Pro Arg Ala Val Glu
            260                 265                 270

Glu Arg Leu Arg Gln His Leu Gln Leu Ala Ser Glu His Leu Ser Ala
        275                 280                 285

Ala Cys Gln His Gly His Ala Thr Gln His Phe Ile Gln Ala Trp Phe
    290                 295                 300

Asp Lys Lys Leu Ala Ala Val Ser
305                 310

<210>  5
<211>  1044
<212>  DNA

<213>  Pantoea agglomerans

<400>  5
atgaaggacg agcgccttgt tcagcgtaag aacgatcatc tggatatcgt tctcgacccc      60

cgtcgcgccg taactcaggc tagcgcaggt tttgagcgct ggcgctttac ccactgcgcc     120

ctgccagagc tgaattttag cgacatcacg ctggaaacca ccttcctgaa tcggcagcta     180

caggctccgc tgctgatcag ctccatgacc ggcggcgttg agcgctcgcg ccatatcaac     240

cgccacctcg ccgaggcggc gcaggtgcta aaaattgcga tgggggtggg ctcccagcgc     300

gtcgccattg agagcgacgc gggcttaggg ctggataaaa ccctgcggca gctggctccg     360

gacgtgccgc tgctggcgaa cctcggcgcg gcgcagctga ccggcagaaa aggtattgat     420

tacgcccgac gggccgtgga gatgatcgag gcggatgcgc tgattgtgca cctaaacccg     480

ctgcaggagg cgctacagcc cggcggcgat cgcgactggc gcggacggct ggcggctatt     540

gaaactctgg tccgcgagct gcccgttccg ctggtggtga aagaggtggg agccggtatc     600

tcccgaaccg tggccgggca gctgatcgat gccggcgtta ccgtgattga cgtcgcgggc     660

gcgggcggca ccagctgggc cgccgttgaa ggcgagcggg cggccaccga gcagcagcgc     720

agcgtggcca acgtctttgc cgactggggg atccccaccg ctgaggcgct ggttgacatt     780

gccgaggcct ggccgcagat gccccttatt gcctcgggcg ggattaaaaa cggcgtcgac     840

gcggcgaaag cgctgcggct cggcgcgtgc atggtagggc aggccgccgc cgtgctcggc     900

agcgcaggcg tctccacgga gaaggtgatc gatcacttca acgtgattat tgagcagctg     960

cgggtggcct gcttctgcac cggcagccgc agcctgagcg atctaaagca ggctgatatc    1020

cgctatgtgc gtgatacgcc ataa                                           1044

<210>  6
<211>  346
<212>  PRT
<213>  Pantoea agglomerans

<400>  6

Met Lys Asp Glu Arg Leu Val Gln Arg Lys Asn Asp His Leu Asp Ile
1               5                   10                  15

Val Leu Asp Pro Arg Arg Ala Val Thr Gln Ala Ser Ala Gly Phe Glu
            20                  25                  30

Arg Trp Arg Phe Thr His Cys Ala Leu Pro Glu Leu Asn Phe Ser Asp
        35                  40                  45

Ile Thr Leu Glu Thr Thr Phe Leu Asn Arg Gln Leu Gln Ala Pro Leu
    50                  55                  60

Leu Ile Ser Ser Met Thr Gly Gly Val Glu Arg Ser Arg His Ile Asn
65                  70                  75                  80

Arg His Leu Ala Glu Ala Ala Gln Val Leu Lys Ile Ala Met Gly Val
                85                  90                  95

Gly Ser Gln Arg Val Ala Ile Glu Ser Asp Ala Gly Leu Gly Leu Asp
            100                 105                 110

Lys Thr Leu Arg Gln Leu Ala Pro Asp Val Pro Leu Leu Ala Asn Leu
        115                 120                 125

Gly Ala Ala Gln Leu Thr Gly Arg Lys Gly Ile Asp Tyr Ala Arg Arg
    130                 135                 140

Ala Val Glu Met Ile Glu Ala Asp Ala Leu Ile Val His Leu Asn Pro
145                 150                 155                 160

Leu Gln Glu Ala Leu Gln Pro Gly Gly Asp Arg Asp Trp Arg Gly Arg
                165                 170                 175

Leu Ala Ala Ile Glu Thr Leu Val Arg Glu Leu Pro Val Pro Leu Val
            180                 185                 190

Val Lys Glu Val Gly Ala Gly Ile Ser Arg Thr Val Ala Gly Gln Leu
        195                 200                 205

Ile Asp Ala Gly Val Thr Val Ile Asp Val Ala Gly Ala Gly Gly Thr
    210                 215                 220

Ser Trp Ala Ala Val Glu Gly Glu Arg Ala Ala Thr Glu Gln Gln Arg
225                 230                 235                 240

Ser Val Ala Asn Val Phe Ala Asp Trp Gly Ile Pro Thr Ala Glu Ala
                245                 250                 255

Leu Val Asp Ile Ala Glu Ala Trp Pro Gln Met Pro Leu Ile Ala Ser
            260                 265                 270

Gly Gly Ile Lys Asn Gly Val Asp Ala Ala Lys Ala Leu Arg Leu Gly
        275                 280                 285

Ala Cys Met Val Gly Gln Ala Ala Ala Val Leu Gly Ser Ala Gly Val
    290                 295                 300

Ser Thr Glu Lys Val Ile Asp His Phe Asn Val Ile Ile Glu Gln Leu
305                 310                 315                 320

Arg Val Ala Cys Phe Cys Thr Gly Ser Arg Ser Leu Ser Asp Leu Lys
                325                 330                 335

Gln Ala Asp Ile Arg Tyr Val Arg Asp Thr
            340                 345

<210>  7
<211>  1362
<212>  DNA
<213>  Picea sitchensis

<400>  7
atgatacagc ggggcccaaa gatgagcaac aaagaagtac acttacctcc ggggagcacg      60

ggttggcctt taataggtga aacgttctcc tactaccgtt ctatgacctc aaatcacccg     120

cgtaagttta ttgacgatcg agagaagcga tatgactcag acatcttcat ctcgcatctc     180

tttggtggtc gtacagttgt ttccgcagat ccgcaattta ataagtttgt ccttcagaat     240

gaaggccgct tcttccaagc gcaatacccg aaggcattaa aggccctaat cggcaactat     300

gggctgctat cagttcacgg cgacctacaa cgaaaattac acggtattgc agtgaacctg     360

ctacgattcg agcgactaaa agtagatttc atggaagaaa ttcagaacct cgtgcacagt     420

acattagata gatgggctga catgaaggaa atttcattgc aaaacgaatg tcaccagatg     480

gtactgaacc tgatggcgaa acagctgctc gatctctcac caagcaagga gacttccgat     540

atatgcgaac tgttcgttga ttatacaaac gccgttatag ccatccctat taagatacct     600

ggctctacgt atgcaaaggg gctaaaagcc agagagttat taatcaagaa gatctccgag     660

atgataaaag agaggaggaa ccatcctgag gtggtgcata acgacctcct aactaagctc     720

gttgaagagg gcctcatcag cgatgaaatc atttgtgact ttatcctctt cctgcttttc     780

gcaggtcatg aaaccagtag tcgcgccatg acattcgcaa ttaagttctt gacatattgc     840

ccaaaagcat taaaacaaat gaaagaggag cacgatgcta tccttaaatc caaaggaggg     900

cataagaagc tgaactggga cgattacaaa tccatggcgt ttactcagtg cgtgattaat     960

gagacactac ggttagggaa cttcgggcca ggagtctttc gtgaagctaa agaagatact    1020

aaagtaaaag actgcttgat ccctaagggt tgggtggtat ttgcctttct taccgccacg    1080

cacctacatg aaaagttcca caatgaagct ctgactttta atccgtggcg ttggcaatta    1140

gacaaagatg ttcccgacga ttctctcttc tcgccctttg gcggaggggc ccgactgtgc    1200

cctggtagtc acttggccaa gctcgagctt agtctgttcc ttcacatatt tatcacacgc    1260

ttctcgtggg aggcgcgagc ggacgatcgg accagctatt tccccttacc ctacttaacg    1320

aagggtttcc ctatatctct gcacggacgc gtagaaaatg aa                       1362

<210>  8

<211>  454
<212>  PRT
<213>  Picea sitchensis

<400>  8

Met Ile Gln Arg Gly Pro Lys Met Ser Asn Lys Glu Val His Leu Pro
1               5                   10                  15

Pro Gly Ser Thr Gly Trp Pro Leu Ile Gly Glu Thr Phe Ser Tyr Tyr
            20                  25                  30

Arg Ser Met Thr Ser Asn His Pro Arg Lys Phe Ile Asp Asp Arg Glu
        35                  40                  45

Lys Arg Tyr Asp Ser Asp Ile Phe Ile Ser His Leu Phe Gly Gly Arg
    50                  55                  60

Thr Val Val Ser Ala Asp Pro Gln Phe Asn Lys Phe Val Leu Gln Asn
65                  70                  75                  80

Glu Gly Arg Phe Phe Gln Ala Gln Tyr Pro Lys Ala Leu Lys Ala Leu
                85                  90                  95

Ile Gly Asn Tyr Gly Leu Leu Ser Val His Gly Asp Leu Gln Arg Lys
            100                 105                 110

Leu His Gly Ile Ala Val Asn Leu Leu Arg Phe Glu Arg Leu Lys Val
        115                 120                 125

Asp Phe Met Glu Glu Ile Gln Asn Leu Val His Ser Thr Leu Asp Arg
    130                 135                 140

Trp Ala Asp Met Lys Glu Ile Ser Leu Gln Asn Glu Cys His Gln Met
145                 150                 155                 160

Val Leu Asn Leu Met Ala Lys Gln Leu Leu Asp Leu Ser Pro Ser Lys
                165                 170                 175

Glu Thr Ser Asp Ile Cys Glu Leu Phe Val Asp Tyr Thr Asn Ala Val
            180                 185                 190

Ile Ala Ile Pro Ile Lys Ile Pro Gly Ser Thr Tyr Ala Lys Gly Leu
        195                 200                 205

Lys Ala Arg Glu Leu Leu Ile Lys Lys Ile Ser Glu Met Ile Lys Glu
    210                 215                 220

Arg Arg Asn His Pro Glu Val Val His Asn Asp Leu Leu Thr Lys Leu

225                 230                 235                 240

Val Glu Glu Gly Leu Ile Ser Asp Glu Ile Ile Cys Asp Phe Ile Leu
                245                 250                 255

Phe Leu Leu Phe Ala Gly His Glu Thr Ser Ser Arg Ala Met Thr Phe
            260                 265                 270

Ala Ile Lys Phe Leu Thr Tyr Cys Pro Lys Ala Leu Lys Gln Met Lys
        275                 280                 285

Glu Glu His Asp Ala Ile Leu Lys Ser Lys Gly Gly His Lys Lys Leu
    290                 295                 300

Asn Trp Asp Asp Tyr Lys Ser Met Ala Phe Thr Gln Cys Val Ile Asn
305                 310                 315                 320

Glu Thr Leu Arg Leu Gly Asn Phe Gly Pro Gly Val Phe Arg Glu Ala
                325                 330                 335

Lys Glu Asp Thr Lys Val Lys Asp Cys Leu Ile Pro Lys Gly Trp Val
            340                 345                 350

Val Phe Ala Phe Leu Thr Ala Thr His Leu His Glu Lys Phe His Asn
        355                 360                 365

Glu Ala Leu Thr Phe Asn Pro Trp Arg Trp Gln Leu Asp Lys Asp Val
    370                 375                 380

Pro Asp Asp Ser Leu Phe Ser Pro Phe Gly Gly Gly Ala Arg Leu Cys
385                 390                 395                 400

Pro Gly Ser His Leu Ala Lys Leu Glu Leu Ser Leu Phe Leu His Ile
                405                 410                 415

Phe Ile Thr Arg Phe Ser Trp Glu Ala Arg Ala Asp Asp Arg Thr Ser
            420                 425                 430

Tyr Phe Pro Leu Pro Tyr Leu Thr Lys Gly Phe Pro Ile Ser Leu His
        435                 440                 445

Gly Arg Val Glu Asn Glu
    450

<210>  9
<211>  1911
<212>  DNA
<213>  Hybrid Zyngiber officinale/Arabidopsis

<400> 9
atggggagga agcccgccga gccgccgaag ccgctggtgg tgaagaccca gatggatgcg 60

gaggaggagg accaagggaa gaagaaggtc accgtcttct tcgggacgca gaccggaacg 120

gctgaggggt tcgcgaaggc gctggctgag gaggcaaagg cacggtaccc taatgccata 180

tttaaagtcg tggatatcga cgaatatgct gctgaggatg atgagtacga ggagaacctg 240

aaaaaggaga gcttggcttt gttcttcttg gctacgtatg gagatggtga gcctactgat 300

aatgctgccc ggttctacaa atggtttaca gaggggaaag agagaggaat ctggttggaa 360

aatcttcaat ttggcgtgtt tggtttgggc aatcggcaat atgaacattt taataaggtt 420

gctgtggtgg tcgatgaact gcttcatgag caaggcgcca agcacattgt ccaagtgggg 480

ttgggagatg atgatcagtg tattgaggat gacttctctg catggaggga gcttctttgg 540

ccggagttgg ataagttgct tcaggatgaa aatgagacag ctgttgccac accatacact 600

gcagctgtgt tagaatacag agtttctatt cacgactctg aagatgccaa attcaatgat 660

ataaacatgg caaatgggaa tggttacact gtgtttgatg ctcaacatcc ttacaaagca 720

aatgtcgctg ttaaaaggga gcttcatact cccgagtctg atcgttcttg tatccatttg 780

gaatttgaca ttgctggaag tggacttacg tatgaaactg gagatcatgt tggtgtactt 840

tgtgataact taagtgaaac tgtagatgaa gctcttagat tgctggatat gtcacctgat 900

acttatttct cacttcacgc tgaaaaagaa gacggcacac caatcagcag ctcactgcct 960

cctcccttcc caccttgcaa cttgagaaca gcgcttacac gatatgcatg tcttttgagt 1020

tctccaaaga agtctgcttt agttgcgttg gctgctcatg catctgatcc taccgaagca 1080

gaacgattaa aacaccttgc ttcacctgct ggaaaggatg aatattcaaa gtgggtagta 1140

gagagtcaaa gaagtctact tgaggtgatg gccgagtttc cttcagccaa gccaccactt 1200

ggtgtcttct tcgctggagt tgctccaagg ttgcagccta ggttctattc gatatcatca 1260

tcgcccaaga ttgctgaaac tagaattcac gtcacatgtg cactggttta tgagaaaatg 1320

ccaactggca ggattcataa gggagtgtgt tccacttgga tgaagaatgc tgtgccttac 1380

gagaagagtg aaaactgttc ctcggcgccg atatttgtta ggcaatccaa cttcaagctt 1440

ccttctgatc ctttagtacc tatcatcatg attggacctg gcacagggtt ggcacctttc 1500

aggggcttct tacaggaaag gttggcattg aaaaatgatg gggtggaact tggtcattct 1560

attctcttct ttggatgcag aaaccgcaaa atggacttca tttacgagga tgagttgaac 1620

aattttgtca aaacaggcgc cctttctgag ttgattgtag ccttctcccg tgagggtcca 1680

actaaacaat atgtgcaaca taaaatgact gagaaagcaa cggaactttg gaatatcatc 1740

tcccaaggcg gatatgtata cgtgtgtgga gatgctaagg gcatggctag agatgttcac 1800

agagttcttc atactattgc tcaagagcag ggaggtatgg atagctccaa aacagaaagc 1860

ttcatcaaga gtttgcaaat ggaagggaga tacctaagag atgtatggtg a          1911

<210>  10
<211>  636
<212>  PRT
<213>  Hybrid Zyngiber officinale/Arabidopsis

<400>  10

Met Gly Arg Lys Pro Ala Glu Pro Pro Lys Pro Leu Val Val Lys Thr
1               5                   10                  15

Gln Met Asp Ala Glu Glu Glu Asp Gln Gly Lys Lys Lys Val Thr Val
            20                  25                  30

Phe Phe Gly Thr Gln Thr Gly Thr Ala Glu Gly Phe Ala Lys Ala Leu
        35                  40                  45

Ala Glu Glu Ala Lys Ala Arg Tyr Pro Asn Ala Ile Phe Lys Val Val
    50                  55                  60

Asp Ile Asp Glu Tyr Ala Ala Glu Asp Asp Glu Tyr Glu Glu Asn Leu
65                  70                  75                  80

Lys Lys Glu Ser Leu Ala Leu Phe Phe Leu Ala Thr Tyr Gly Asp Gly
                85                  90                  95

Glu Pro Thr Asp Asn Ala Ala Arg Phe Tyr Lys Trp Phe Thr Glu Gly
            100                 105                 110

Lys Glu Arg Gly Ile Trp Leu Glu Asn Leu Gln Phe Gly Val Phe Gly
        115                 120                 125

Leu Gly Asn Arg Gln Tyr Glu His Phe Asn Lys Val Ala Val Val Val
    130                 135                 140

Asp Glu Leu Leu His Glu Gln Gly Ala Lys His Ile Val Gln Val Gly
145                 150                 155                 160

Leu Gly Asp Asp Asp Gln Cys Ile Glu Asp Asp Phe Ser Ala Trp Arg
                165                 170                 175

Glu Leu Leu Trp Pro Glu Leu Asp Lys Leu Leu Gln Asp Glu Asn Glu
            180                 185                 190

Thr Ala Val Ala Thr Pro Tyr Thr Ala Ala Val Leu Glu Tyr Arg Val
        195                 200                 205

Ser Ile His Asp Ser Glu Asp Ala Lys Phe Asn Asp Ile Asn Met Ala
    210                 215                 220

Asn Gly Asn Gly Tyr Thr Val Phe Asp Ala Gln His Pro Tyr Lys Ala
225                 230                 235                 240

Asn Val Ala Val Lys Arg Glu Leu His Thr Pro Glu Ser Asp Arg Ser
                245                 250                 255

Cys Ile His Leu Glu Phe Asp Ile Ala Gly Ser Gly Leu Thr Tyr Glu
            260                 265                 270

Thr Gly Asp His Val Gly Val Leu Cys Asp Asn Leu Ser Glu Thr Val
        275                 280                 285

Asp Glu Ala Leu Arg Leu Leu Asp Met Ser Pro Asp Thr Tyr Phe Ser
    290                 295                 300

Leu His Ala Glu Lys Glu Asp Gly Thr Pro Ile Ser Ser Ser Leu Pro
305                 310                 315                 320

Pro Pro Phe Pro Pro Cys Asn Leu Arg Thr Ala Leu Thr Arg Tyr Ala
                325                 330                 335

Cys Leu Leu Ser Ser Pro Lys Lys Ser Ala Leu Val Ala Leu Ala Ala
            340                 345                 350

His Ala Ser Asp Pro Thr Glu Ala Glu Arg Leu Lys His Leu Ala Ser
        355                 360                 365

Pro Ala Gly Lys Asp Glu Tyr Ser Lys Trp Val Val Glu Ser Gln Arg
    370                 375                 380

Ser Leu Leu Glu Val Met Ala Glu Phe Pro Ser Ala Lys Pro Pro Leu
385                 390                 395                 400

Gly Val Phe Phe Ala Gly Val Ala Pro Arg Leu Gln Pro Arg Phe Tyr
                405                 410                 415

Ser Ile Ser Ser Ser Pro Lys Ile Ala Glu Thr Arg Ile His Val Thr
            420                 425                 430

Cys Ala Leu Val Tyr Glu Lys Met Pro Thr Gly Arg Ile His Lys Gly
        435                 440                 445

Val Cys Ser Thr Trp Met Lys Asn Ala Val Pro Tyr Glu Lys Ser Glu
    450                 455                 460

Asn Cys Ser Ser Ala Pro Ile Phe Val Arg Gln Ser Asn Phe Lys Leu
465                 470                 475                 480

Pro Ser Asp Pro Leu Val Pro Ile Ile Met Ile Gly Pro Gly Thr Gly
                485                 490                 495

Leu Ala Pro Phe Arg Gly Phe Leu Gln Glu Arg Leu Ala Leu Lys Asn
            500                 505                 510

Asp Gly Val Glu Leu Gly His Ser Ile Leu Phe Phe Gly Cys Arg Asn
        515                 520                 525

Arg Lys Met Asp Phe Ile Tyr Glu Asp Glu Leu Asn Asn Phe Val Lys
    530                 535                 540

Thr Gly Ala Leu Ser Glu Leu Ile Val Ala Phe Ser Arg Glu Gly Pro
545                 550                 555                 560

Thr Lys Gln Tyr Val Gln His Lys Met Thr Glu Lys Ala Thr Glu Leu
                565                 570                 575

Trp Asn Ile Ile Ser Gln Gly Gly Tyr Val Tyr Val Cys Gly Asp Ala
            580                 585                 590

Lys Gly Met Ala Arg Asp Val His Arg Val Leu His Thr Ile Ala Gln
        595                 600                 605

Glu Gln Gly Gly Met Asp Ser Ser Lys Thr Glu Ser Phe Ile Lys Ser
    610                 615                 620

Leu Gln Met Glu Gly Arg Tyr Leu Arg Asp Val Trp
625                 630                 635

<210>  11
<211>  7693
<212>  DNA
<213>  pGT1695

<400>  11
acccagtttc atcattccat tttattttgc gagcgagcgc acacttgtga attatctcaa      60

tagcagtgtg aaataacata attgagcaac tgaaagggag tgcccaatat tacgacatca     120

tccatcacgc ggagttacct gattatgaca cagagggccc accatcacca tcaccattcc     180

atgatacagc ggggcccaaa gatgagcaac aaagaagtac acttacctcc ggggagcacg     240

ggttggcctt taataggtga aacgttctcc tactaccgtt ctatgacctc aaatcacccg     300

cgtaagttta ttgacgatcg agagaagcga tatgactcag acatcttcat ctcgcatctc     360

tttggtggtc gtacagttgt ttccgcagat ccgcaattta ataagtttgt ccttcagaat 420

gaaggccgct tcttccaagc gcaatacccg aaggcattaa aggccctaat cggcaactat 480

gggctgctat cagttcacgg cgacctacaa cgaaaattac acggtattgc agtgaacctg 540

ctacgattcg agcgactaaa agtagatttc atggaagaaa ttcagaacct cgtgcacagt 600

acattagata gatgggctga catgaaggaa atttcattgc aaaacgaatg tcaccagatg 660

gtactgaacc tgatggcgaa acagctgctc gatctctcac caagcaagga gacttccgat 720

atatgcgaac tgttcgttga ttatacaaac gccgttatag ccatccctat taagatacct 780

ggctctacgt atgcaaaggg gctaaaagcc agagagttat taatcaagaa gatctccgag 840

atgataaaag agaggaggaa ccatcctgag gtggtgcata acgacctcct aactaagctc 900

gttgaagagg gcctcatcag cgatgaaatc atttgtgact ttatcctctt cctgcttttc 960

gcaggtcatg aaaccagtag tcgcgccatg acattcgcaa ttaagttctt gacatattgc 1020

ccaaaagcat taaaacaaat gaaagaggag cacgatgcta tccttaaatc caaaggaggg 1080

cataagaagc tgaactggga cgattacaaa tccatggcgt ttactcagtg cgtgattaat 1140

gagacactac ggttagggaa cttcgggcca ggagtctttc gtgaagctaa agaagatact 1200

aaagtaaaag actgcttgat ccctaagggt tgggtggtat ttgcctttct taccgccacg 1260

cacctacatg aaaagttcca caatgaagct ctgactttta atccgtggcg ttggcaatta 1320

gacaaagatg ttcccgacga ttctctcttc tcgccctttg gcggaggggc ccgactgtgc 1380

cctggtagtc acttggccaa gctcgagctt agtctgttcc ttcacatatt tatcacacgc 1440

ttctcgtggg aggcgcgagc ggacgatcgg accagctatt tccccttacc ctacttaacg 1500

aagggtttcc ctatatctct gcacggacgc gtagaaaatg aaatgtccgg ttctgggatg 1560

gggaggaagc ccgccgagcc gccgaagccg ctggtggtga agacccagat ggatgcggag 1620

gaggaggacc aagggaagaa gaaggtcacc gtcttcttcg ggacgcagac cgggacggct 1680

gaggggttcg cgaaggcgct ggctgaggag gcaaaggcac ggtaccctaa tgccatattt 1740

aaagtcgtgg atatcgacga atatgctgct gaggatgatg agtacgagga gaacctgaaa 1800

aaggagagct tggctttgtt cttcttggct acgtatggag atggtgagcc tactgataat 1860

gctgcccggt tctacaaatg gtttacagag gggaaagaga gaggaatctg gttggaaaat 1920

cttcaatttg gcgtgtttgg tttgggcaat cggcaatatg aacattttaa taaggttgct 1980

gtggtggtcg atgaactgct tcatgagcaa ggcgccaagc acattgtcca agtggggttg 2040

ggagatgatg atcagtgtat tgaggatgac ttctctgcat ggagggagct tctttggccg 2100

gagttggata agttgcttca ggatgaaaat gagacagctg ttgccacacc atacactgca 2160

gctgtgttag aatacagagt ttctattcac gactctgaag atgccaaatt caatgatata 2220

aacatggcaa atgggaatgg ttacactgtg tttgatgctc aacatcctta caaagcaaat 2280

gtcgctgtta aaagggagct tcatactccc gagtctgatc gttcttgtat ccatttggaa 2340

tttgacattg ctggaagtgg acttacgtat gaaactggag atcatgttgg tgtactttgt 2400

gataacttaa gtgaaactgt agatgaagct cttagattgc tggatatgtc acctgatact 2460

tatttctcac ttcacgctga aaaagaagac ggcacaccaa tcagcagctc actgcctcct 2520

cccttcccac cttgcaactt gagaacagcg cttacacgat atgcatgtct tttgagttct 2580

ccaaagaagt ctgctttagt tgcgttggct gctcatgcat ctgatcctac cgaagcagaa 2640

cgattaaaac accttgcttc acctgctgga aaggatgaat attcaaagtg ggtagtagag 2700

agtcaaagaa gtctacttga ggtgatggcc gagtttcctt cagccaagcc accacttggt 2760

gtcttcttcg ctggagttgc tccaaggttg cagcctaggt tctattcgat atcatcatcg 2820

cccaagattg ctgaaactag aattcacgtc acatgtgcac tggtttatga gaaaatgcca 2880

actggcagga ttcataaggg agtgtgttcc acttggatga agaatgctgt gccttacgag 2940

aagagtgaaa actgttcctc ggcgccgata tttgttaggc aatccaactt caagcttcct 3000

tctgatcctt tagtacctat catcatgatt ggacctggca cagggttggc acctttcagg 3060

ggcttcttac aggaaaggtt ggcattgaaa aatgatgggg tggaacttgg tcattctatt 3120

ctcttctttg gatgcagaaa ccgcaaaatg gacttcattt acgaggatga gttgaacaat 3180

tttgtcaaaa caggcgccct ttctgagttg attgtagcct tctcccgtga gggtccaact 3240

aaacaatatg tgcaacataa aatgactgag aaagcaacgg aactttggaa tatcatctcc 3300

caaggcggat atgtatacgt gtgtggagat gctaagggca tggctagaga tgttcacaga 3360

gttcttcata ctattgctca agagcaggga ggtatggata gctccaaaac agaaagcttc 3420

atcaagagtt tgcaaatgga agggagatac ctaagagatg tatggtgagg atccggctgc 3480

taacaaagcc cgaaaggaag ctgagttggc tgctgccacc gctgagcaat aactagcata 3540

accccttggg gcctctaaac gggtcttgag gggttttttg ctgaaaggag gaactatatc 3600

cggccggata tccacaggac gggtgtggtc gccatgatcg cgtagtcgat agtggctcca 3660

agtagcgaag cgagcaggac tgggcggcgg ccaaagcggt cggacagtgc tccgagaacg 3720

ggtgcgcata gaaattgcat caacgcatat agcgctagca gcacgccata gtgactggcg 3780

atgctgtcgg aatggacgat atcccgcaag aggcccggca gtaccggcat aaccaagcct 3840

atgcctacag catccagggt gacggtgccg aggatgacga tgagcgcatt gttagatttc 3900

atacacggtg cctgactgcg ttagcaattt aactgtgata aactaccgca ttaaagctta 3960

tcgatgataa gctgtcaaac atgagaattc ttgaagacga aagggcctcg tgatacgcct 4020

atttttatag gttaatgtca tgcatgagac aataaccctg ataaatgctt caataatatt 4080

gaaaaaggaa gagtatgagt attcaacatt tccgtgtcgc ccttattccc ttttttgcgg 4140

cattttgcct tcctgttttt gctcacccag aaacgctggt gaaagtaaaa gatgctgaag 4200

atcagttggg tgcacgagtg ggttacatcg aactggatct caacagcggt aagatccttg 4260

agagttttcg ccccgaagaa cgttttccaa tgatgagcac ttttaaagtt ctgctatgtg 4320

gcgcggtatt atcccgtgtt gacgccgggc aagagcaact cggtcgccgc atacactatt 4380

ctcagaatga cttggttgac gcgtcaccag tcacagaaaa gcatcttacg gatggcatga 4440

cagtaagaga attatgcagt gctgccataa ccatgagtga taacactgcg gccaacttac 4500

ttctgacaac gatcggagga ccgaaggagc taaccgcttt tttgcacaac atgggggatc 4560

atgtaactcg ccttgatcgt tgggaaccgg agctgaatga agccatacca aacgacgagc 4620

gtgacaccac gatgcctgca gcaatggcaa caacgttgcg caaactatta actggcgaac 4680

tacttactct agcttcccgg caacaattaa tagactggat ggaggcggat aaagttgcag 4740

gaccacttct gcgctcggcc cttccggctg gctggtttat tgctgataaa tctggagccg 4800

gtgagcgtgg gtctcgcggt atcattgcag cactggggcc agatggtaag ccctcccgta 4860

tcgtagttat ctacacgacg gggagtcagg caactatgga tgaacgaaat agacagatcg 4920

ctgagatagg tgcctcactg attaagcatt ggtaactgtc agaccaagtt tactcatata 4980

tactttagat tgatttaaaa cttcattttt aatttaaaag gatctaggtg aagatccttt 5040

ttgataatct catgcatgac caaaatccct taacgtgagt tttcgttcca ctgagcgtca 5100

gaccccgtag aaaagatcaa aggatcttct tgagatcctt tttttctgcg cgtaatctgc 5160

tgcttgcaaa caaaaaaacc accgctacca gcggtggttt gtttgccgga tcaagagcta 5220

ccaactcttt ttccgaaggt aactggcttc agcagagcgc agataccaaa tactgtcctt 5280

ctagtgtagc cgtagttagg ccaccacttc aagaactctg tagcaccgcc tacatacctc 5340

gctctgctaa tcctgttacc agtggctgct gccagtggcg ataagtcgtg tcttaccggg 5400

ttggactcaa gacgatagtt accggataag gcgcagcggt cgggctgaac ggggggttcg 5460

tgcacacagc ccagcttgga gcgaacgacc tacaccgaac tgagatacct acagcgtgag 5520

ctatgagaaa gcgccacgct tcccgaaggg agaaaggcgg acaggtatcc ggtaagcggc 5580

agggtcggaa caggagagcg cacgagggag cttccagggg gaaacgcctg gtatctttat 5640

agtcctgtcg ggtttcgcca cctctgactt gagcgtcgat ttttgtgatg ctcgtcaggg 5700

gggcggagcc tatggaaaaa cgccagcaac gcggcctttt tacggttcct ggccttttgc 5760

tggccttttg ctcacatgtt ctttcctgcg ttatcccctg attctgtgga taaccgtatt 5820

accgcctttg agtgagctga taccgctcgc cgcagccgaa cgaccgagcg cagcgagtca 5880

gtgagcgagg aagcggaaga gcgcctgatg cggtattttc tccttacgca tctgtgcggt 5940

atttcacacc gcatatatgg tgcactctca gtacaatctg ctctgatgcc gcatagttaa 6000

gccagtatac actccgctat cgctacgtga ctgggtcatg gctgcgcccc gacacccgcc 6060

aacacccgct gacgcgccct gacgggcttg tctgctcccg gcatccgctt acagacaagc 6120

tgtgaccgtc tccgggagct gcatgtgtca gaggttttca ccgtcatcac cgaaacgcgc 6180

gaggcagctg gcacgacagg tttcccgact gggttggtgc gttttatcat gcctggcggg 6240

taggtcggat aaggcgttca cgccgcatcc gacaaccacg cagcgttacc tgatgtgacg 6300

ccgacaattc tcatcatcgc tacaacatga cctcgctatt tacatcgcga tactcttttg 6360

gcgtcgtgtc atatgctttt ttaaaaacag agtagaaata ttgcagcgat ggataaccgc 6420

acatttgcga tatctcattg atcgacaagg tggttgaaat cagcagactg cgcgctttct 6480

ccagcttctc ggcatgaatc atggcatgga tggtttcacc cacctcttct ttaaaacgct 6540

tctcaagatt ggagcgcgag atcccgaccg catccagtac ctgatccact ttaatccctt 6600

tacaggcgtg attacgaatg taatgcatgg cctgaataac ggcgggatcg gtcagcgagc 6660

gataatctgt tgagcgccgt tcaatgacgc gaactggtgg gaccaaaatt cgctgtagcg 6720

gcatttcttc tttatctaat aatcgatgca acagttttgc cgcctgatag cccatttgcc 6780

gcgcgccctg agcgaccgaa gaaagggcga cacgcgacag atagcgggtc agttcttcgt 6840

tatcgatgcc aatcacgcat aatttttccg gtacgggaat atgtagatgt tcacatactt 6900

gcagaatatg ccgcgctcgg gcgtcagtaa cggcaataat cccggtttgc ggtggtagcg 6960

tttgtagcca gtctgccagc cgattttgcg cgtgttgcca gttctctggc gcggtttcta 7020

acccctgata aaccactccg cgatactttt cttcggcgac aagctgacga aatgcatatt 7080

cgcgctcagt ggcccaacgt ttgccgcttg attccggaag accataaaaa gcaaagcggt 7140

taacgccttt ctcttttaaa tgcaaaaatg cgctttcaac cagcgcatag ttatcggtgg 7200

caatgtaatg aacgggtggg taactttctg caaggtgata cgagccgcca accccaacaa 7260

tggggacgtc gacatcagcc agcgcttgct cgatctgttt gtcgtcgaag tcggcaatga 7320

cgccatctcc taaccagtcc ttgattttat caatgcgggc gcggaaatct tcttcaatga 7380

aaatatccca ttccgattgt gacgcctgta aatattcccc tacgccttct actacctgcc 7440

ggtcataggc tttattggca ttgaacagta atgtgatgcg gtgacgttta gtaaacatgg 7500

ttcttttcct gctgaatcat gctaagtaac aatcaccgcg ataaacgtaa ccaattttta 7560

gcaactaaac aggggaaaac aattacagat ttttatcttt cgattacgat ttttggttta 7620

tttcttgatt tatgaccgag atcttacttt tgttgcgcaa ttgtacttat tgcatttttc 7680

tcttcgagga att 7693

<210> 12
<211> 6779
<212> DNA
<213> pGT1681

<400> 12
gagtgtatac tggcttacta tgttggcact gatgagggtg tcagtgaagt gcttcatgtg 60

gcaggagaaa aaaggctgca ccggtgcgtc agcagaatat gtgatacagg atatattccg 120

cttcctcgct cactgactcg ctacgctcgg tcgttcgact gcggcgagcg gaaatggctt 180

acgaacgggg cggagatttc ctggaagatg ccaggaagat acttaacagg gaagtgagag 240

ggccgcggca aagccgtttt tccataggct ccgcccccct gacaagcatc acgaaatctg 300

acgctcaaat cagtggtggc gaaacccgac aggactataa agataccagg cgtttccccc 360

tggcggctcc ctcgtgcgct ctcctgttcc tgcctttcgg tttaccggtg tcattccgct 420

gttatggccg cgtttgtctc attccacgcc tgacactcag ttccgggtag gcagttcgct 480

ccaagctgga ctgtatgcac gaaccccccg ttcagtccga ccgctgcgcc ttatccggta 540

actatcgtct tgagtccaac ccggaaagac atgcaaaagc accactggca gcagccactg 600

gtaattgatt tagaggagtt agtcttgaag tcatgcgccg gttaaggcta aactgaaagg 660

acaagttttg gtgactgcgc tcctccaagc cagttacctc ggttcaaaga gttggtagct 720

cagagaacct tcgaaaaacc gccctgcaag gcggtttttt cgttttcaga gcaagagatt 780

acgcgcagac caaaacgatc tcaagaagat catcttatta atcagataaa atatttctag 840

atttcagtgc aatttatctc ttcaaatgta gcacctgaag tcagccccat acgatataag 900

ttgtaattct catgtttgac agcttatcat cgataagctt taatgcggta gtttatcaca 960

gttaaattgc taacgcagtc aggaaccttg caatgcatta tccgtttata aggacagccc 1020

gaatgacggt ctgcgcaaaa aaacacgttc atctcactcg cgatgctgcg gagcagttac 1080

tggctgatat tgatcgacgc cttgatcagt tattgcccgt ggagggagaa cgggatgttg 1140

tgggtgccgc gatgcgtgaa ggtgcgctgg caccgggaaa acgtattcgc cccatgttgc 1200

tgttgctgac cgcccgcgat ctgggttgcg ctgtcagcca tgacggatta ctggatttgg 1260

cctgtgcggt ggaaatggtc cacgcggctt cgctgatcct tgacgatatg ccctgcatgg 1320

acgatgcgaa gctgcggcgc ggacgcccta ccattcattc tcattacgga gagcatgtgg 1380

caatactggc ggcggttgcc ttgctgagta aagcctttgg cgtaattgcc gatgcagatg 1440

gcctcacgcc gctggcaaaa aatcgggcgg tttctgaact gtcaaacgcc atcggcatgc 1500

aaggattggt tcagggtcag ttcaaggatc tgtctgaagg ggataagccg cgcagcgctg 1560

aagctatttt gatgacgaat cactttaaaa ccagcacgct gttttgtgcc tccatgcaga 1620

tggcctcgat tgttgcgaat gcctccagcg aagcgcgtga ttgcctgcat cgtttttcac 1680

ttgatcttgg tcaggcattt caactgctgg acgatttgac cgatggcatg accgacaccg 1740

gtaaggatag caatcaggac gccggtaaat cgacgctggt caatctgtta ggcccgaggg 1800

cggttgaaga acgtctgaga caacatcttc agcttgccag tgagcatctc tctgcggcct 1860

gccaacacgg gcacgccact caacatttta ttcaggcctg gtttgacaaa aaactcgctg 1920
ccgtcagtta ataccgccct tttgggttca agcagtacat aacgatggaa ccacattaca 1980
ggagtagtga tgaatgaagg acgagcgcct tgttcagcgt aagaacgatc atctggatat 2040
cgttctcgac ccccgtcgcg ccgtaactca ggctagcgca ggttttgagc gctggcgctt 2100
tacccactgc gccctgccag agctgaattt tagcgacatc acgctggaaa ccaccttcct 2160
gaatcggcag ctacaggctc cgctgctgat cagctccatg accggcggcg ttgagcgctc 2220
gcgccatatc aaccgccacc tcgccgaggc ggcgcaggtg ctaaaaattg cgatgggggt 2280
gggctcccag cgcgtcgcca ttgagagcga cgcgggctta gggctggata aaaccctgcg 2340
gcagctggct ccggacgtgc cgctgctggc gaacctcggc gcggcgcagc tgaccggcag 2400
aaaaggtatt gattacgccc gacgggccgt ggagatgatc gaggcggatg cgctgattgt 2460
gcacctaaac ccgctgcagg aggcgctaca gcccggcggc gatcgcgact ggcgcggacg 2520
gctggcggct attgaaactc tggtccgcga gctgcccgtt ccgctggtgg tgaaagaggt 2580
gggagccggt atctcccgaa ccgtggccgg gcagctgatc gatgccggcg ttaccgtgat 2640
tgacgtcgcg ggcgcgggcg gcaccagctg ggccgccgtt gaaggcgagc gggcggccac 2700
cgagcagcag cgcagcgtgg ccaacgtctt tgccgactgg gggatcccca ccgctgaggc 2760
gctggttgac attgccgagg cctggccgca gatgcccctt attgcctcgg gcgggattaa 2820
aaacggcgtc gacgcggcga aagcgctgcg gctcggcgcg tgcatggtag ggcaggccgc 2880
cgccgtgctc ggcagcgcag gcgtctccac ggagaaggtg atcgatcact tcaacgtgat 2940
tattgagcag ctgcgggtgg cctgcttctg caccggcagc cgcagcctga gcgatctaaa 3000
gcaggctgat atccgctatg tgcgtgatac gccataagga ggtacaacca tgacagcggc 3060
agagacacgg ccggaccagc tgccgcaaga agaaaggttc gtaagtcgcc taaatgccga 3120
ttatcaccct gccgtgtgga aggacgactt tattgatagt ttaaccagcc cgaacagcca 3180
tgcaacctcc aaatcgtctg ttgatgaaac tatcaataaa cgtatccaga ccttggtgaa 3240
agaaatacaa tgcatgtttc agagcatggg cgatggtgaa acgaatcctt ctgcttacga 3300
taccgcgtgg gtggcaagaa taccgtcgat cgacggcagt ggcgcaccgc agtttcccca 3360
gacacttcag tggattttaa acaatcagct gccggatggt agctggggtg aagaatgcat 3420
ctttttggca tatgatcgag tgctgaatac gttagcttgc ttgcttacgc tcaagatctg 3480
gaacaaaggc gatattcaag ttcagaaagg cgtcgagttc gtacgtaaac atatggagga 3540
gatgaaagat gaggccgaca atcatcggcc ttccgggttc gaggtggtat ttccggccat 3600
gttagatgaa gctaagagcc ttggcctgga cctgccatac catctgcctt ttattagcca 3660
gatccaccaa aaacggcaaa agaaattaca gaaaattccg ttaaatgttt tacataacca 3720
tcagacagcg cttttatact ctctcgaggg gctgcaggat gttgtcgatt ggcaggaaat 3780

tactaatctt caatcgcgtg atggaagttt tctgagcagt ccggcttcca ccgcttgtgt 3840

cttcatgcat acacaaaaca aacgctgttt gcatttcctg aactttgtgt tatctaagtt 3900

cggtgactat gtaccttgtc actacccgtt ggatctcttt gaacgcctgt gggcggttga 3960

cacggtagaa cgtctcggca tcgataggta ttttaaaaaa gaaattaaag aaagccttga 4020

ttatgtgtac cgttactggg acgcagaacg aggagtgggc tgggcgcgat gtaacccgat 4080

tccagatgtc gatgacaccg caatggggct gcgaatccta cgcctgcatg gctataacgt 4140

cagctccgac gtcctggaga atttccggga tgaaaagggc gacttctttt gcttcgcggg 4200

ccagactcag atagggggta ccgataatct gaatctgtac cgttgtagtc aggtgtgctt 4260

tcctggtgaa aaaattatgg aagaagcgaa gacgtttacg actaaccacc tgcaaaacgc 4320

cctggccaaa aataacgcat tcgataagtg ggctgtcaag aaggatctgc cgggcgaagt 4380

agaatacgcc attaagtatc cctggcatcg ctccatgccg cgtctggaag cccgttccta 4440

tatcgagcag tttggttcaa atgacgtctg gctcggtaaa acagtataca aaatgctgta 4500

tgttagcaac gagaaatatc ttgagctggc caaactggat ttcaatatgg ttcaagcttt 4560

gcaccaaaag gaaacgcaac acattgtgtc atggtggcgc gagtctggct ttaatgatct 4620

gacgtttact cgtcagcgtc cggtagaaat ctatttctcc gtggcggtga gtatgttcga 4680

accagagttc gccgcttgtc gcattgccta cgcaaaaacc tcatgcctcg cggtgatcct 4740

ggacgatctg tatgatacgc atggtagtct ggatgacctc aaattgttta gcgaggcggt 4800

ccgccgttgg gatattagtg ttcttgacag cgtgcgcgat aaccaactga aggtgtgttt 4860

tctcggactg ttcaacacag tgaacggttt cggcaaagat gggctgaaag aacagggccg 4920

cgatgtcttg ggttatttgc gcaaggtgtg ggaaggtttg ctagcgtcct acacgaaaga 4980

ggcggaatgg agcgcagcaa agtatgtgcc aacctttaat gaatatgtcg agaatgcaaa 5040

agtttcaatt gcgctcgcca ccgtagttct gaattcaatc ttctttaccg gcgagctgct 5100

gccagattac attctccagc aggtagacct gcgctcgaaa ttcttacatc tggtgagtct 5160

caccggccgt ttgataaacg ataccaaaac ataccaggcc gaacgtaacc gcggggagct 5220

ggtttcttcg gtccaatgtt acatgcgcga gaacccggag tgtactgaag aggaagctct 5280

cagccacgtc tacggcataa ttgacaacgc actgaaggaa ctgaactggg agctagcaaa 5340

ccctgcgtcc aacgcgccct tatgcgtgcg ccgtctgttg tttaataccg ctcgtgtcat 5400

gcagcttttt tacatgtatc gtgacggttt tggaatttcg gataaagaaa tgaaagatca 5460

cgtgtcccgt accttatttg acccggtagc atgaggatcc ggctgctaac aaagcccgtt 5520

ctgtttaaga acgggatttt ttgctgaaag gaggaactat atccggccgg atatcccgcg 5580

cgcttgtttc ggcgtgggta tggtggcagg ccccgtggcc gggggactgt tgggcgccat 5640

ctccttgggg tcgaatttgc tttcgaattt ctgccattca tccgcttatt atcacttatt 5700

caggcgtagc accaggcgtt taagggcacc aataactgcc ttaaaaaaat tacgccccgc 5760

cctgccactc atcgcagtac tgttgtaatt cattaagcat tctgccgaca tggaagccat 5820

cacagacggc atgatgaacc tgaatcgcca gcggcatcag caccttgtcg ccttgcgtat 5880

aatatttgcc catggtgaaa acgggggcga agaagttgtc catattggcc acgtttaaat 5940

caaaactggt gaaactcacc cagggattgg ctgagacgaa aaacatattc tcaataaacc 6000

ctttagggaa ataggccagg ttttcaccgt aacacgccac atcttgcgaa tatatgtgta 6060

gaaactgccg gaaatcgtcg tggtattcac tccagagcga tgaaaacgtt tcagtttgct 6120

catggaaaac ggtgtaacaa gggtgaacac tatcccatat caccagctca ccgtctttca 6180

ttgccatacg gaattccgga tgagcattca tcaggcgggc aagaatgtga ataaaggccg 6240

gataaaactt gtgcttattt ttctttacgg tctttaaaaa ggccgtaata tccagctgaa 6300

cggtctggtt ataggtacat tgagcaactg actgaaatgc ctcaaaatgt tctttacgat 6360

gccattggga tatatcaacg gtggtatatc cagtgatttt tttctccatt ttagcttcct 6420

tagctcctga aaatctcgat aactcaaaaa atacgcccgg tagtgatctt atttcattat 6480

ggtgaaagtt ggaacctctt acgtgccgat caacgtctca ttttcgccaa aagttggccc 6540

agggcttccc ggtatcaaca gggacaccag gatttattta ttctgcgaag tgatcttccg 6600

tcacaggtat ttattcggcg caaagtgcgt cgggtgatgc tgccaactta ctgatttagt 6660

gtatgatggt gtttttgagg tgctccagtg gcttctgttt ctatcagctg tccctcctgt 6720

tcagctactg acggggtggt gcgtaacggc aaaagcaccg ccggacatca gcgctagcg 6779

<210> 13
<211> 708
<212> DNA
<213> Curcuma wenyujin

<400> 13
atggcagccg tagccgacga tgccagcatg gatgctgttc agcgccgtct tatgtttgaa 60

gatgaatgta ttctcgttga cgaaaacgac gttgtgattg gacatgagtc gaaatataat 120

tgccatcttg tcgaaaagat tgagtcgttg aacctcttgc accgtgcgtt ttcagtgttt 180

ctgtttaata gcaaatatga attactttta cagcaacgca gtgaaaccaa ggtgaccttc 240

cccctggtct ggacaaacac ctgctgcagc caccccttgt accgcgagtc tgagttaatt 300

caagaaaact acctgggtgt gcgcaatgcc gcgcagcgta aactcctgga tgaactgggc 360

attcctgcgg aggacattcc agtggatgag tttatcccgc tgggccgtat gctgtacaag 420

gcgccatccg acggtaaatg gggcgagcat gaggtagatt atttgctgtt tatcgtgcgc 480

gatgtgaaac tgctgccgaa tccggatgaa gtggcggatg taaagtatgt caatcgtgat 540

cagctgaaag agctgctgcg caaggcggat gccggagagg aaggtgtgaa actgtcacct 600

tggttccgct tggtcgtgga caactttctg atggaatggt gggcgcgtgt ggaacagggt 660

accctgctgg aggcagccga tatggaaaca atccataagc tggtctaa 708

<210> 14
<211> 235
<212> PRT
<213> Curcuma wenyujin

<400> 14

Met Ala Ala Val Ala Asp Asp Ala Ser Met Asp Ala Val Gln Arg Arg
1               5                   10                  15

Leu Met Phe Glu Asp Glu Cys Ile Leu Val Asp Glu Asn Asp Val Val
            20                  25                  30

Ile Gly His Glu Ser Lys Tyr Asn Cys His Leu Val Glu Lys Ile Glu
        35                  40                  45

Ser Leu Asn Leu Leu His Arg Ala Phe Ser Val Phe Leu Phe Asn Ser
    50                  55                  60

Lys Tyr Glu Leu Leu Leu Gln Gln Arg Ser Glu Thr Lys Val Thr Phe
65                  70                  75                  80

Pro Leu Val Trp Thr Asn Thr Cys Cys Ser His Pro Leu Tyr Arg Glu
                85                  90                  95

Ser Glu Leu Ile Gln Glu Asn Tyr Leu Gly Val Arg Asn Ala Ala Gln
            100                 105                 110

Arg Lys Leu Leu Asp Glu Leu Gly Ile Pro Ala Glu Asp Ile Pro Val
        115                 120                 125

Asp Glu Phe Ile Pro Leu Gly Arg Met Leu Tyr Lys Ala Pro Ser Asp
    130                 135                 140

Gly Lys Trp Gly Glu His Glu Val Asp Tyr Leu Leu Phe Ile Val Arg
145                 150                 155                 160

Asp Val Lys Leu Leu Pro Asn Pro Asp Glu Val Ala Asp Val Lys Tyr
                165                 170                 175

Val Asn Arg Asp Gln Leu Lys Glu Leu Leu Arg Lys Ala Asp Ala Gly
            180                 185                 190

Glu Glu Gly Val Lys Leu Ser Pro Trp Phe Arg Leu Val Val Asp Asn

195                 200                 205

Phe Leu Met Glu Trp Trp Ala Arg Val Glu Gln Gly Thr Leu Leu Glu
    210                 215                 220

Ala Ala Asp Met Glu Thr Ile His Lys Leu Val
225                 230                 235

<210>  15
<211>  2394
<212>  DNA
<213>  Abies grandis

<400>  15
atgagcgctg aaaagaatga ttcgctttcg tcgtcaactc tggtaaagcg agagttcccg      60

ccgggattct ggaaagacga cttgatcgac tcgcttacca gcagtcacaa agtggccgct     120

agtgacgaaa agcgcataga aactcttatc agtgaaatta agaatatgtt ccgttgtatg     180

ggatatggag aaaccaatcc cagtgcatac gacacggcat gggtggctcg aattccagca     240

gtagatggtt ccgataatcc tcactttcca gaaacagttg aatggatttt acaaaaccag     300

ctaaaagatg ggtcatgggg tgaaggtttc tacttcctcg cttatgaccg gattctagcc     360

actttagctt gtatcattac actcacgcta tggcgcacag gggaaaccca ggtacagaag     420

ggtattgagt tcttccggac ccaagcaggc aagatggaag atgaggcaga ttctcaccgt     480

ccgtccgggt tcgagatagt cttccccgct atgttgaaag aggctaagat tctgggcttg     540

gacctgccat acgacctccc tttcctcaag cagattatcg agaagagaga agctaaactg     600

aaacgtatac ctaccgatgt cttatatgcc ttgcctacga cccttttgta ctcgttggag     660

ggactccaag aaatcgtgga ctggcaaaag attatgaaat tgcaatccaa agacggctca     720

ttcttgtcaa gccccgcgtc gactgctgca gtgtttatgc gtaccggcaa caagaaatgt     780

ttagacttct taaatttcgt actgaagaag ttcggtaacc atgtgccgtg ccattacccg     840

ttggatcttt tcgagcggtt gtgggctgtc gacactgttg agaggctcgg aatagaccgt     900

catttcaagg aggagataaa agaggctctc gactacgtat attctcattg ggatgaacgc     960

ggcataggtt gggcgagaga aaatccagtc ccggacatag acgacaccgc aatgggcctc    1020

agaatcctgc gcctacacgg gtacaatgtg tcgtctgatg tgctgaaaac tttccgcgac    1080

gaaaatgggg aattcttctg tttcctaggt cagacccagc gaggagtgac cgatatgttg    1140

aacgtcaatc ggtgcagtca tgtgtccttt ccaggcgaga ctatcatgga agaagccaag    1200

ctctgtactg aacggtacct ccgaaacgcg ctcgagaatg tagatgcatt tgacaaatgg    1260

gcattcaaga agaacattcg aggagaggtt gagtacgcct taaagtatcc gtggcataag    1320

tcgatgccta ggctagaggc ccggtcgtac atagagaact atggtccgga tgatgtctgg    1380

ttgggcaaga ctgtgtacat gatgccctat attagcaacg aaaagtatct ggagttagcc 1440

aaactagact ttaataaggt tcaaagcatc caccaaacgg agctgcaaga tcttagacgt 1500

tggtggaaga gcagtgggtt caccgatctc aatttcacgc gagaacgtgt cactgagata 1560

tattttagcc ctgcctcgtt catcttcgaa cccgaattct ctaaatgtag ggaggtttac 1620

acaaagacaa gtaactttac tgttatactg gacgacctat acgacgcgca cgggagtctt 1680

gacgacctga agctgttcac tgagagtgtg aaacgctggg acctgagtct cgtcgaccaa 1740

atgccgcagc agatgaagat ctgctttgtg ggcttctata atacttttaa cgacattgcg 1800

aaggagggca gggaacgcca aggacgtgac gtactgggat acattcagaa cgtctggaaa 1860

gtacaactcg aggcatatac aaaggaggct gaatggtcgg aggctaagta tgttccgtct 1920

ttcaacgaat acatcgaaaa cgcatcggtg tcaatagcct tgggcacggt agtcctaatc 1980

tcggctcttt tcactgggga agtcttgacc gatgaggttc tctcaaagat tgaccgggaa 2040

tcgagatttc ttcaattgat gggtctcacc ggtcgattag tcaacgatac gaagacgtat 2100

caagcagaga ggggtcaggg tgaagtagct agcgctattc aatgttacat gaaggaccat 2160

cccaagatat ccgaggagga ggctcttcag catgtatact cagtcatgga gaacgctctc 2220

gaagagttaa acagagaatt tgtcaacaat aagatccctg acatctacaa gaggctggtg 2280

ttcgaaaccg ctcgcatcat gcagctattc tacatgcaag gcgatggtct caccctttcc 2340

catgacatgg aaataaagga gcacgttaag aactgcctat tccagccagt cgcc 2394

<210> 16
<211> 798
<212> PRT
<213> Abies grandis

<400> 16

Met Ser Ala Glu Lys Asn Asp Ser Leu Ser Ser Ser Thr Leu Val Lys
1               5                   10                  15

Arg Glu Phe Pro Pro Gly Phe Trp Lys Asp Asp Leu Ile Asp Ser Leu
            20                  25                  30

Thr Ser Ser His Lys Val Ala Ala Ser Asp Glu Lys Arg Ile Glu Thr
        35                  40                  45

Leu Ile Ser Glu Ile Lys Asn Met Phe Arg Cys Met Gly Tyr Gly Glu
    50                  55                  60

Thr Asn Pro Ser Ala Tyr Asp Thr Ala Trp Val Ala Arg Ile Pro Ala
65                  70                  75                  80

Val Asp Gly Ser Asp Asn Pro His Phe Pro Glu Thr Val Glu Trp Ile

                85                  90                  95

Leu Gln Asn Gln Leu Lys Asp Gly Ser Trp Gly Glu Gly Phe Tyr Phe
            100                 105                 110

Leu Ala Tyr Asp Arg Ile Leu Ala Thr Leu Ala Cys Ile Ile Thr Leu
        115                 120                 125

Thr Leu Trp Arg Thr Gly Glu Thr Gln Val Gln Lys Gly Ile Glu Phe
    130                 135                 140

Phe Arg Thr Gln Ala Gly Lys Met Glu Asp Glu Ala Asp Ser His Arg
145                 150                 155                 160

Pro Ser Gly Phe Glu Ile Val Phe Pro Ala Met Leu Lys Glu Ala Lys
                165                 170                 175

Ile Leu Gly Leu Asp Leu Pro Tyr Asp Leu Pro Phe Leu Lys Gln Ile
            180                 185                 190

Ile Glu Lys Arg Glu Ala Lys Leu Lys Arg Ile Pro Thr Asp Val Leu
        195                 200                 205

Tyr Ala Leu Pro Thr Thr Leu Leu Tyr Ser Leu Glu Gly Leu Gln Glu
    210                 215                 220

Ile Val Asp Trp Gln Lys Ile Met Lys Leu Gln Ser Lys Asp Gly Ser
225                 230                 235                 240

Phe Leu Ser Ser Pro Ala Ser Thr Ala Ala Val Phe Met Arg Thr Gly
                245                 250                 255

Asn Lys Lys Cys Leu Asp Phe Leu Asn Phe Val Leu Lys Lys Phe Gly
            260                 265                 270

Asn His Val Pro Cys His Tyr Pro Leu Asp Leu Phe Glu Arg Leu Trp
        275                 280                 285

Ala Val Asp Thr Val Glu Arg Leu Gly Ile Asp Arg His Phe Lys Glu
    290                 295                 300

Glu Ile Lys Glu Ala Leu Asp Tyr Val Tyr Ser His Trp Asp Glu Arg
305                 310                 315                 320

Gly Ile Gly Trp Ala Arg Glu Asn Pro Val Pro Asp Ile Asp Asp Thr
                325                 330                 335

Ala Met Gly Leu Arg Ile Leu Arg Leu His Gly Tyr Asn Val Ser Ser
        340                 345                 350

Asp Val Leu Lys Thr Phe Arg Asp Glu Asn Gly Glu Phe Phe Cys Phe
    355                 360                 365

Leu Gly Gln Thr Gln Arg Gly Val Thr Asp Met Leu Asn Val Asn Arg
    370                 375                 380

Cys Ser His Val Ser Phe Pro Gly Glu Thr Ile Met Glu Glu Ala Lys
385                 390                 395                 400

Leu Cys Thr Glu Arg Tyr Leu Arg Asn Ala Leu Glu Asn Val Asp Ala
                405                 410                 415

Phe Asp Lys Trp Ala Phe Lys Lys Asn Ile Arg Gly Glu Val Glu Tyr
        420                 425                 430

Ala Leu Lys Tyr Pro Trp His Lys Ser Met Pro Arg Leu Glu Ala Arg
    435                 440                 445

Ser Tyr Ile Glu Asn Tyr Gly Pro Asp Asp Val Trp Leu Gly Lys Thr
    450                 455                 460

Val Tyr Met Met Pro Tyr Ile Ser Asn Glu Lys Tyr Leu Glu Leu Ala
465                 470                 475                 480

Lys Leu Asp Phe Asn Lys Val Gln Ser Ile His Gln Thr Glu Leu Gln
                485                 490                 495

Asp Leu Arg Arg Trp Trp Lys Ser Ser Gly Phe Thr Asp Leu Asn Phe
        500                 505                 510

Thr Arg Glu Arg Val Thr Glu Ile Tyr Phe Ser Pro Ala Ser Phe Ile
    515                 520                 525

Phe Glu Pro Glu Phe Ser Lys Cys Arg Glu Val Tyr Thr Lys Thr Ser
    530                 535                 540

Asn Phe Thr Val Ile Leu Asp Asp Leu Tyr Asp Ala His Gly Ser Leu
545                 550                 555                 560

Asp Asp Leu Lys Leu Phe Thr Glu Ser Val Lys Arg Trp Asp Leu Ser
                565                 570                 575

Leu Val Asp Gln Met Pro Gln Gln Met Lys Ile Cys Phe Val Gly Phe
        580                 585                 590

Tyr Asn Thr Phe Asn Asp Ile Ala Lys Glu Gly Arg Glu Arg Gln Gly
        595                 600                 605

Arg Asp Val Leu Gly Tyr Ile Gln Asn Val Trp Lys Val Gln Leu Glu
    610                 615                 620

Ala Tyr Thr Lys Glu Ala Glu Trp Ser Glu Ala Lys Tyr Val Pro Ser
625                 630                 635                 640

Phe Asn Glu Tyr Ile Glu Asn Ala Ser Val Ser Ile Ala Leu Gly Thr
                645                 650                 655

Val Val Leu Ile Ser Ala Leu Phe Thr Gly Glu Val Leu Thr Asp Glu
            660                 665                 670

Val Leu Ser Lys Ile Asp Arg Glu Ser Arg Phe Leu Gln Leu Met Gly
        675                 680                 685

Leu Thr Gly Arg Leu Val Asn Asp Thr Lys Thr Tyr Gln Ala Glu Arg
    690                 695                 700

Gly Gln Gly Glu Val Ala Ser Ala Ile Gln Cys Tyr Met Lys Asp His
705                 710                 715                 720

Pro Lys Ile Ser Glu Glu Glu Ala Leu Gln His Val Tyr Ser Val Met
                725                 730                 735

Glu Asn Ala Leu Glu Glu Leu Asn Arg Glu Phe Val Asn Asn Lys Ile
            740                 745                 750

Pro Asp Ile Tyr Lys Arg Leu Val Phe Glu Thr Ala Arg Ile Met Gln
        755                 760                 765

Leu Phe Tyr Met Gln Gly Asp Gly Leu Thr Leu Ser His Asp Met Glu
    770                 775                 780

Ile Lys Glu His Val Lys Asn Cys Leu Phe Gln Pro Val Ala
785                 790                 795

<210>  17
<211>  2358
<212>  DNA
<213>  Pinus contorta

<400>  17
atggccaagc gcgaagagat tccgcccggc gtatggaagg acgacatctc tgatagcatt      60

atgagttcac ataagccaga agccgatgaa aaacgtgtcg aaatcctgat tgcggagatt     120

aaatcaatgt ttcgcgggat gggagatggc gagaccactc cgtcagccta tgatacggcg 180

tgggtggcca agattcctgc gctggatggt tcggaccacc ctcactttcc gcaaaccctg 240

cagtggattc tccagaacca attacaagac ggaagctggg gcgaaggtac ctatttctcc 300

acgtatgacc ggctcctcgc gaccttagcc tgtatcatta cgctcacagt ctggcgcact 360

ggccagaccc aggtgcgaca gggcattgag tttttccgca aacatgcagg gactatggag 420

gatgaggcgg ataaccatca accgatagga tttgaattcg tttttcccgc gatgattaat 480

gaagccaaaa gtctgtgtct ggatctgccg tacgacaccc ctttcatcaa acaaattatc 540

gaaaagcgcg aagcgaaatt gaaaatgatt ccgacggata cgctgtacac ggtgcctact 600

acgttcctct actacttaga agggctacaa gagatcatcg actgccaaaa aataattaag 660

ttgcaaagca aagatggttc atttttgagt agtcccgcct cgaccgcggc ggtttttatg 720

tgcaccggca ataccaagtg tctcgagttt cttaactttg tgctgattaa gttcggtaat 780

cacgtgccgt gccaataccc gctggattta tttgaaagat tatgggcagt tgatatcgtg 840

gaacgcctgg gcattgaccg ccatttcaaa aaagagatta aggatgcatt agattacgtc 900

tatagtcatt gggacgaacg tgggattcga tgggcacgag aaaacccagt cgcgtatatc 960

gatgttatgg ccacaggcat tcgtatactg aggctgcatc gttacaacgt gagctctgac 1020

atcctgaaaa cctttcgaga tgaaaacgga gagttttatc gcttcccagg ccaatccgag 1080

cggggtgtta ccgatatgct gaacttgaac cgctgcagcc acgtggcatt tccaggtgaa 1140

actgtcatgg aggaagcaaa gttatgtaca gaaagatacc tgtggaacgc gttggagaat 1200

gtcaatcccc tggacaagtg gggtctgaag gagaatatcc gcggtgaagt tgagtatgcg 1260

ttgaaatatc cgtggcttcg ccgtttgccc cgtttggaaa cgcgtaacta catcgagcat 1320

tacgggcaaa acgatgtgtg gctgggcaag atgatgcaca tgatgcccta catcaatgat 1380

cgcaaatacc tggagctcgc taaattggac tttaataatg tacagagcat acatcaaaag 1440

gaactgcgcg aattgcgtcg ttggtggaag tcatctggtt ttgccgaact taactttctg 1500

ccagaccgag tcgccgagat cttcttctcc atcgcaagtt caatgttcga accggaactg 1560

gctacctgcc gcgcggtata cactaaatcc acactgtgca ctgtcattct ggacggcttc 1620

tatgacgttc acgggtcggc cgaagacatt atgttattca atgaagcggt caaacgctgg 1680

gatcactctc tgctcgatcg tatggccgaa catatcaaaa cgtgtttctt tgctctgtac 1740

aacgttgtta acgaaattgc ggaagagggt cgtaagcgtc agggccatga cgtgctgccg 1800

tatattcgta atttatggga aatccagctt gaatctttca cgaaagaagc agaatggtcg 1860

agagctgaac atgtgccgtc cttccatgaa tacatagagg ccgctgccat atcatcagca 1920

ctgccaactc tcgttctgat tggcgttatt ttcactggag aggttctcac agatcacata 1980

ctctcccaaa ttgattaccg atccaaattt gcatatctca tgagcttgac agggcgtttg 2040

gcgaacgata ccaaaactta ccaggtggag agaggtcaag gagaggtggc ttctgccata 2100

cagtgttaca tgaaggaaaa tcccgaattg tcagaggaag aagctctcga atatatttat 2160

aggctcatgg aaaacgccct cgcagatttc aagtgtgaat ttctgaatac caaagacgtg 2220

ccggagtatt gcagaagact agtttttgac aatgcgagat caatgcagct gatttacatg 2280

gaaggagacg gtttcaaact ttcacatgaa acggaaatta aacagcatgt caaaaagatc 2340

ctgttcgaac cagttgcc 2358

<210> 18
<211> 786
<212> PRT
<213> Pinus contorta

<400> 18

Met Ala Lys Arg Glu Glu Ile Pro Pro Gly Val Trp Lys Asp Asp Ile
1               5                   10                  15

Ser Asp Ser Ile Met Ser Ser His Lys Pro Glu Ala Asp Glu Lys Arg
            20                  25                  30

Val Glu Ile Leu Ile Ala Glu Ile Lys Ser Met Phe Arg Gly Met Gly
        35                  40                  45

Asp Gly Glu Thr Thr Pro Ser Ala Tyr Asp Thr Ala Trp Val Ala Lys
    50                  55                  60

Ile Pro Ala Leu Asp Gly Ser Asp His Pro His Phe Pro Gln Thr Leu
65                  70                  75                  80

Gln Trp Ile Leu Gln Asn Gln Leu Gln Asp Gly Ser Trp Gly Glu Gly
                85                  90                  95

Thr Tyr Phe Ser Thr Tyr Asp Arg Leu Leu Ala Thr Leu Ala Cys Ile
            100                 105                 110

Ile Thr Leu Thr Val Trp Arg Thr Gly Gln Thr Gln Val Arg Gln Gly
        115                 120                 125

Ile Glu Phe Phe Arg Lys His Ala Gly Thr Met Glu Asp Glu Ala Asp
    130                 135                 140

Asn His Gln Pro Ile Gly Phe Glu Phe Val Phe Pro Ala Met Ile Asn
145                 150                 155                 160

Glu Ala Lys Ser Leu Cys Leu Asp Leu Pro Tyr Asp Thr Pro Phe Ile
                165                 170                 175

Lys Gln Ile Ile Glu Lys Arg Glu Ala Lys Leu Lys Met Ile Pro Thr
            180                 185                 190

Asp Thr Leu Tyr Thr Val Pro Thr Thr Phe Leu Tyr Tyr Leu Glu Gly
        195                 200                 205

Leu Gln Glu Ile Ile Asp Cys Gln Lys Ile Ile Lys Leu Gln Ser Lys
    210                 215                 220

Asp Gly Ser Phe Leu Ser Ser Pro Ala Ser Thr Ala Ala Val Phe Met
225                 230                 235                 240

Cys Thr Gly Asn Thr Lys Cys Leu Glu Phe Leu Asn Phe Val Leu Ile
                245                 250                 255

Lys Phe Gly Asn His Val Pro Cys Gln Tyr Pro Leu Asp Leu Phe Glu
            260                 265                 270

Arg Leu Trp Ala Val Asp Ile Val Glu Arg Leu Gly Ile Asp Arg His
        275                 280                 285

Phe Lys Lys Glu Ile Lys Asp Ala Leu Asp Tyr Val Tyr Ser His Trp
    290                 295                 300

Asp Glu Arg Gly Ile Arg Trp Ala Arg Glu Asn Pro Val Ala Tyr Ile
305                 310                 315                 320

Asp Val Met Ala Thr Gly Ile Arg Ile Leu Arg Leu His Arg Tyr Asn
                325                 330                 335

Val Ser Ser Asp Ile Leu Lys Thr Phe Arg Asp Glu Asn Gly Glu Phe
            340                 345                 350

Tyr Arg Phe Pro Gly Gln Ser Glu Arg Gly Val Thr Asp Met Leu Asn
        355                 360                 365

Leu Asn Arg Cys Ser His Val Ala Phe Pro Gly Glu Thr Val Met Glu
    370                 375                 380

Glu Ala Lys Leu Cys Thr Glu Arg Tyr Leu Trp Asn Ala Leu Glu Asn
385                 390                 395                 400

Val Asn Pro Leu Asp Lys Trp Gly Leu Lys Glu Asn Ile Arg Gly Glu
                405                 410                 415

Val Glu Tyr Ala Leu Lys Tyr Pro Trp Leu Arg Arg Leu Pro Arg Leu

            420                 425                 430

Glu Thr Arg Asn Tyr Ile Glu His Tyr Gly Ala Asn Asp Val Trp Leu
        435                 440                 445

Gly Lys Met Met His Met Met Pro Tyr Ile Asn Asp Arg Lys Tyr Leu
    450                 455                 460

Glu Leu Ala Lys Leu Asp Phe Asn Asn Val Gln Ser Ile His Gln Lys
465                 470                 475                 480

Glu Leu Arg Glu Leu Arg Arg Trp Trp Lys Ser Ser Gly Phe Ala Glu
                485                 490                 495

Leu Asn Phe Leu Pro Asp Arg Val Ala Glu Ile Phe Phe Ser Ile Ala
            500                 505                 510

Ser Ser Met Phe Glu Pro Glu Leu Ala Thr Cys Arg Ala Val Tyr Thr
        515                 520                 525

Lys Ser Thr Leu Cys Thr Val Ile Leu Asp Gly Phe Tyr Asp Val His
    530                 535                 540

Gly Ser Ala Glu Asp Ile Met Leu Phe Asn Glu Ala Val Lys Arg Trp
545                 550                 555                 560

Asp His Ser Leu Leu Asp Arg Met Ala Glu His Ile Lys Thr Cys Phe
                565                 570                 575

Leu Ala Leu Tyr Asn Val Val Asn Glu Ile Ala Glu Glu Gly Arg Lys
            580                 585                 590

Arg Gln Gly His Asp Val Leu Pro Tyr Ile Arg Asn Leu Trp Glu Ile
        595                 600                 605

Gln Leu Glu Ser Phe Thr Lys Glu Ala Glu Trp Ser Arg Ala Glu His
    610                 615                 620

Val Pro Ser Phe His Glu Tyr Ile Glu Ala Ala Ala Ile Ser Ser Ala
625                 630                 635                 640

Leu Pro Thr Leu Val Leu Ile Gly Val Ile Phe Thr Gly Glu Val Leu
                645                 650                 655

Thr Asp His Ile Leu Ser Gln Ile Asp Tyr Arg Ser Lys Phe Ala Tyr
            660                 665                 670

Leu Met Ser Leu Thr Gly Arg Leu Ala Asn Asp Thr Lys Thr Tyr Gln
        675                 680                 685

Val Glu Arg Gly Gln Gly Glu Val Ala Ser Ala Ile Gln Cys Tyr Met
    690                 695                 700

Lys Glu Asn Pro Glu Leu Ser Glu Glu Glu Ala Leu Glu Tyr Ile Tyr
705                 710                 715                 720

Arg Leu Met Glu Asn Ala Leu Ala Asp Phe Lys Cys Glu Phe Leu Asn
                725                 730                 735

Thr Lys Asp Val Pro Glu Tyr Cys Arg Arg Leu Val Phe Asp Asn Ala
            740                 745                 750

Arg Ser Met Gln Leu Ile Tyr Met Glu Gly Asp Gly Phe Lys Leu Ser
        755                 760                 765

His Glu Thr Glu Ile Lys Gln His Val Lys Lys Ile Leu Phe Glu Pro
    770                 775                 780

Val Ala
785

## Beschreibung

### Technisches Gebiet der Erfindung

Die vorliegende Erfindung betrifft einen rekombinanten Mikroorganismus zur fermentativen *de novo* Synthese von Harzsäuregemischen definierter Zusammensetzung, wobei der rekombinante Mikroorganismus die Enzymaktivitäten wenigstens einer Geranylgeranyl-Pyrophosphat-Synthase (GGPPS), wenigstens einer Isopentenyl-Pyrophosphat-Isomerase (IPI), wenigstens einer Diterpensynthase (diTPS), wenigstens einer Cytochrom-P450-Monooxygenase (CYP), und wenigstens einer Cytochrom-P450-Reduktase (CPR) aufweist, wobei die Zusammensetzung im Wesentlichen durch die diTPS bestimmt wird.

Weiterhin betrifft die Erfindung ein Verfahren zur fermentativen *de novo* Synthese von Harzsäuregemischen definierter Zusammensetzung, umfassend die Kultivierung des rekombinanten Mikroorganismus.

Die vorliegende Erfindung betrifft auch die Verwendung des rekombinanten Mikroorganismus zur fermentativen *de novo* Synthese von Harzsäuregemischen definierter Zusammensetzung.

### Hintergrund der Erfindung/Stand der Technik

Naturharze werden schon seit dem Altertum für verschiedene Zwecke eingesetzt, unter anderem auch zum Kleben und als Dichtstoffe. Der jährliche Bedarf der chemischen Industrie in Deutschland an Naturharzen wird auf 31.000 Tonnen geschätzt. Die überwiegende Menge wird zur Herstellung von Farben, Lacken und Klebstoffen eingesetzt. Ausgehend von Naturharzen werden hierzu Harzsäuregemische aus dem Naturstoff gewonnen und für die verschiedenen Anwendungen eingesetzt, wobei die Quelle des Naturharzes die Zusammensetzung des abgeleiteten Harzsäuregemischs bestimmt, was naturgemäß saisonalen Schwankungen unterliegt.

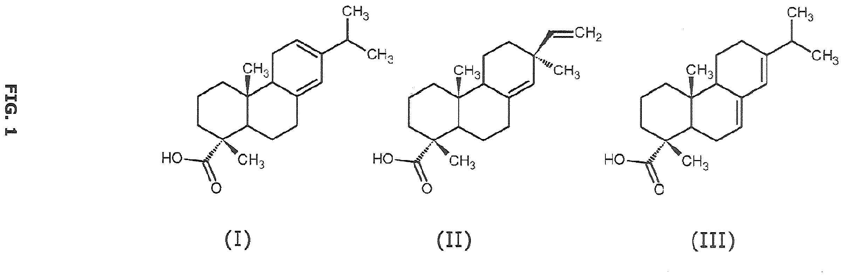

Harzsäuren bilden die Hauptbestandteile in natürlichen Harzen, wie beispielsweise dem aus Balsam (dickflüssiges, sirupartiges Gemisch aus Harzen und ätherischen Ölen) von Kiefern, Fichten und Tannen hergestellten Kolophonium. Kolophonium ist ein Sammelbegriff für aus Koniferen gewonnene, natürliche Harze, die hauptsächlich aus einem Gemisch verschiedener Harzsäuren bestehen. In dieser Form findet es allerdings technisch kaum Verwendung, da Kolophonium als Naturstoff zum einen stark schwankenden Eigenschaften unterliegt, und zum anderen nicht alterungsbeständig ist. Daher werden bislang aus Kolophonium durch Destillation die reinen Harzsäuren gewonnen, die auf verschiedene Arten weiterverarbeitet werden können. Typische Vertreter von Harzsäuren sind beispielsweise Abietinsäure und Pimarsäure (FIG. 1). Chemisch zählen die Harzsäuren zu den Carbonsäuren und gehören der Naturstoffklasse der Diterpene an.

Im Klebstoffbereich werden für Schmelzklebstoffe nahezu ausschließlich die Ester der Harzsäuren eingesetzt. Im Bereich der Lösemittelklebstoffe kommen jedoch auch überwiegend die nicht veresterten Harze zum Einsatz. Die bei der thermischen Veresterung stattfindende Umisomerisierung zum stabilsten Isomer (der Abietinsäure; FIG. 1) unterbleibt dementsprechend bei einer solchen Anwendung. Insbesondere bei der Verwendung in Lösemittelklebstoffen sind die Isomerengemische wichtig für die Funktion, da eine Reinsubstanz zu schnell und vollständig kristallisieren könnte, was negativen Einfluss auf die Hafteigenschaften haben kann.

Aus industrieller/wirtschaftlicher Sicht ist es sinnvoll und wünschenswert neue Wege zu finden, um (natürliche) Schwankungen der Produktqualität zu minimieren oder ganz zu vermeiden und um zunehmend auftretende Engpässe bei der Rohstoffversorgung zu umgehen. Darüber hinaus ist es wünschenswert, die Abhängigkeit von Rohstoffimporten zu minimieren und zugleich die Nutzung heimischer Ressourcen zu stärken. Ein geeignetes fermentatives System bietet die Aussicht, diese Punkte ideal zu verknüpfen. Eine kontrollierte und standardisierte Harzsäureproduktion könnte unter Verwendung von Zucker als Ausgangsmaterial, der aus heimischen Quellen, beispielsweise aus Zuckerrüben oder über die Verzuckerung von Lignocellulose, bereitgestellt werden könnte, erfolgen. Auch zuckerhaltige Restströme, wie sie bei verschiedensten industriellen Prozessen anfallen, könnten grundsätzlich als Ausgangsmaterial einer fermentativen Harzsäuresynthese dienen.

### Harzsäure-Biosynthese

Gemeinsame Ausgangsverbindungen in der Biosynthese der Terpene sind das Isopentenyldiphosphat (IPP) und das durch Isomerisierung aus IPP gebildete Dimethylallyldiphosphat (DMAPP), die in der Pflanze über den cytosolischen MVA-Weg oder den in den Chloroplasten lokalisierten MEP-Syntheseweg gebildet werden (Kirby und Keasling, Annu. Rev. Plant Biol. 60:335-355 (2009)). Aus diesen Metaboliten werden die Ausgangsverbindungen der verschiedenen Terpenklassen erzeugt: Geranyldiphosphat (GPP), Farnesyldiphosphat (FPP) und Geranylgeranyldiphosphat (GGPP). Letzteres ist dabei gemeinsamer Grundstoff für die Biosynthese nicht nur der Diterpene, sondern auch der Tetraterpene (z.B. Carotinoide). Die jeweiligen Schlüsselenzyme sind die Diterpensynthase (diTPS) bzw. die Phytoensynthase für die Tetraterpene. Die diTPS formt GGPP in das zyklische Zwischenprodukt Copalyl-Pyrophosphat (CPP) um, das dann über mehrere Oxidationsschritte zum Diterpen-Endprodukt umgesetzt wird (Lafever u.a., Arch. Biochem. Biophys. 313: 139-149 (1994)), während die Phytoensynthase zwei Moleküle GGPP zu Phytoen verknüpft. Da beide Enzyme in Chloroplasten lokalisiert sind, liegt hier eine Konkurrenzreaktion vor und die relativen Aktivitäten der Schlüsselenzyme beeinflussen/steuern den Flux aus dem vorliegenden GGPP - Pool in die beiden unterschiedlichen Stoffwechselwege.

Über die Realisierung eines synthetischen Stoffwechselwegs in geeigneten mikrobiellen Wirten könnte diese Konkurrenzreaktion vermieden werden, was, neben der prinzipbedingten Vermeidung unerwünschter Nebenprodukte, im Idealfall auch höhere Produktausbeuten verspräche. Voraussetzung hierfür ist selbstverständlich die Bereitstellung der benötigten Ausgangsverbindungen durch den Stoffwechsel des Wirtsorganismus. In bakteriellen Wirten ist der MEP-Weg in der Regel vorhanden, sodass die benötigten Grundsubstanzen IPP und DMAPP in diesen Zellen verfügbar sind. Dies gilt insbesondere auch für das häufig verwendete Bakterium *E. coli.* Allerdings verfügt *E. coli* über keine Enzymfunktionen, um das für die Diterpen-Synthese benötigte GGPP herzustellen, so dass es im Normalfall nicht bereitsteht (Toyomasu, Biosci. Biotechnol. Biochem. 72: 1168-1175 (2008)). Durch Einbau und rekombinante Expression geeigneter Enzymfunktionen sind bereits rekombinante *E. coli*-Stämme etabliert worden, die zur Bildung von GGPP befähigt sind. Mit ihrer Hilfe konnten bislang synthetische Stoffwechselwege zur fermentativen Produktion von Carotinoiden (Tetraterpenen) erfolgreich etabliert werden (Cunningham, Plant Cell 8: 1613-1626 (1996)).

Im Pflanzenreich sind zwei Typen von Diterpensynthasen vertreten: monofunktionale und bifunktionale Enzyme (Toyomasu, Biosci. Biotechnol. Biochem. 72: 1168-1175 (2008)). Dementsprechend wird die Bildung der Dien-Zwischenverbindungen aus denen dann die Harzsäuren erzeugt werden über zwei sukzessiv arbeitende, monofunktionale Enzyme oder ein bifunktionales Enzym katalysiert. Bifunktionale Enzymvarianten sind insbesondere für Koniferen beschrieben und charakterisiert worden (Vogel u.a., J. Biol. Chem. 271: 23262-23268 (1996)). Die Wahl der verwendeten Diterpensynthase bestimmt die synthetisierbare Harzsäure.

Die Oxidation der Diterpensynthase-Produkte zu den Harzsäuren erfolgt mittels Enzymen, die der Klasse der Cytochrom-P450-Monooxygenasen (P450-Enzyme) angehören. Hier sind bereits geeignete Enzyme beschrieben worden, die interessanterweise alle drei notwendigen Oxidationen (zum Alkohol, zum Aldehyd und dann zur Säure) katalysieren können (Ro u.a., Proc. Natl. Acad. Sci. USA 102: 8060-8065 (2005)). Die Verwertung der Dien-Zwischenverbindungen könnte wünschenswerterweise durch die Identifizierung alternativer P450-Enzyme mit besserer Substratspezifität, oder die Identifizierung und Einbindung eines zusätzlichen, Dien-spezifischen P450-Enzyms optimiert werden.

Aus technischer Sicht ist außerdem zu berücksichtigen, dass P450-Enzyme Cofaktor-abhängig sind, NAD(P)H für ihre katalytische Aktivität benötigen und zumeist auf die Wechselwirkung mit externen Redoxproteinen (Cytochrom P450-Oxidoreduktasen, CPR) angewiesen sind (Jensen und Møller, Phytochemistry 71: 132-141 (2010)). Dabei konnte in den vergangenen Jahren gezeigt werden, dass die Redoxpartner einer größeren Variabilität unterliegen können als bis dato vermutet (Taylor und King, J. Chromatogr. Sci. 39: 269-272 (2001)). Es ist aber grundsätzlich möglich P450-Reduktasen erfolgreich mit unterschiedlichen P450-Enzymen zu verwenden.

Eine Aufgabe der vorliegenden Erfindung ist somit die Reindarstellung einer gewünschten Harzsäure über ein fermentatives Verfahren. Dieses bietet die Aussicht, je nach verwendeter Enzymkombination, unterschiedliche, Harzsäuregemische zu bilden, die je nach verwendeter Diterpensynthase eine Harzsäure als Hauptkomponente enthalten. Solche Gemische mit vorbestimmten adhäsiven und sonstigen gewünschten Eigenschaften könnten dann für den jeweiligen Anwendungszweck optimiert werden.

### Kurzbeschreibung der Erfindung

Erfindungsgemäß wurde überraschend gefunden, dass Harzsäuren und Harzsäuregemische definierter Zusammensetzung in einem fermentativen Verfahren *de novo* synthetisiert werden können.
Dieses neuartige Verfahren erlaubt es erstmals Harzsäuren wie Abietinsäure, Pimarsäure und Levopimarsäure als einzelne Verbindungen zu erzeugen. Diese Substanzen sind in dieser Form und als einzelne Substanzen bislang am Markt nicht erhältlich. Außerdem erlaubt die Verfügbarkeit der reinen Harzsäuren durch gezielte Zumischung der fermentativ gewonnenen Präparate in vorhandene Harzsäuremischungen eine kontinuierlich hohe Qualität zu sichern.

Die vorliegende Erfindung betrifft dabei die folgenden Aspekte:
(1) einen rekombinanten Mikroorganismus zur fermentativen *de novo* Synthese von Harzsäuregemischen definierter Zusammensetzung, wobei der rekombinante Mikroorganismus die Enzymaktivitäten (i) wenigstens einer Geranylgeranyl-Pyrophosphat-Synthase (GGPPS), (ii) wenigstens einer Isopentenyl-Pyrophosphat-Isomerase (IPI), (iii) wenigstens einer Diterpensynthase (diTPS), welche die Zusammensetzung bestimmt, (iv) wenigstens einer Cytochrom-P450-Monooxygenase (CYP), und (v) wenigstens einer Cytochrom-P450-Reduktase (CPR) aufweist;
(2) ein Verfahren zur fermentativen *de novo* Synthese von Harzsäuregemischen definierter Zusammensetzung, umfassend die Kultivierung eines rekombinanten Mikroorganismus entsprechend obigem Aspekt (1); und
(3) die Verwendung eines rekombinanten Mikroorganismus entsprechend obigem Aspekt (1) in einem Verfahren gemäß obigem Aspekt (2).
Weitere, bevorzugte Aspekte der Erfindung sind in den abhängigen Ansprüchen beschrieben.

### Kurzbeschreibung der Figuren

Figur 1: Chemische Strukturen der Harzsäuren. Die Abbildung zeigt die Struktur der erfindungsgemäß fermentativ herstellbaren Harzsäure Levopimarsäure (I) im Vergleich zu den verwandten Strukturen der Pimarsäure (II) und der Abietinsäure (III).

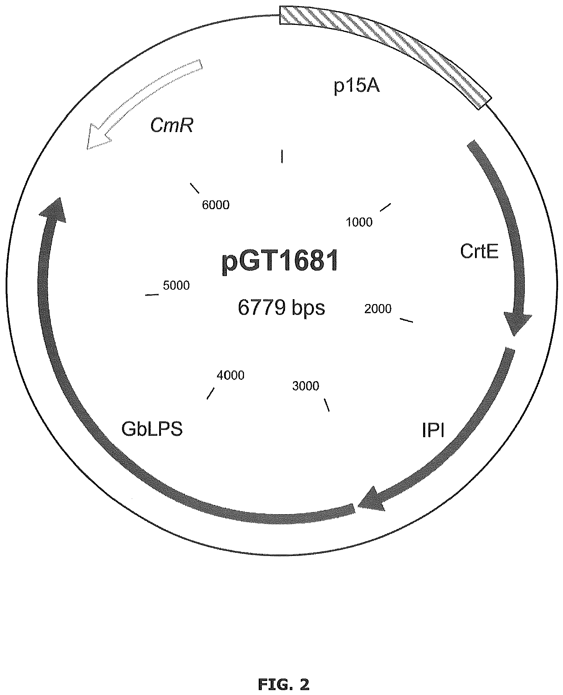

Figur 2: Plasmid enthaltend die Enzymkaskade zur Biosynthese der Grundstruktur Levopimaradien, mit crtE: Geranylgeranyl-diphosphat-Synthase; IPI: Isopentenyl-Diphosphat-Isomerase; GbLPS: Terpensynthase Levopimardiensynthase; CmR: Chloramphenicol-Resistenzgen; p15A: Regulator der Plasmidreplikation (origin of replication).

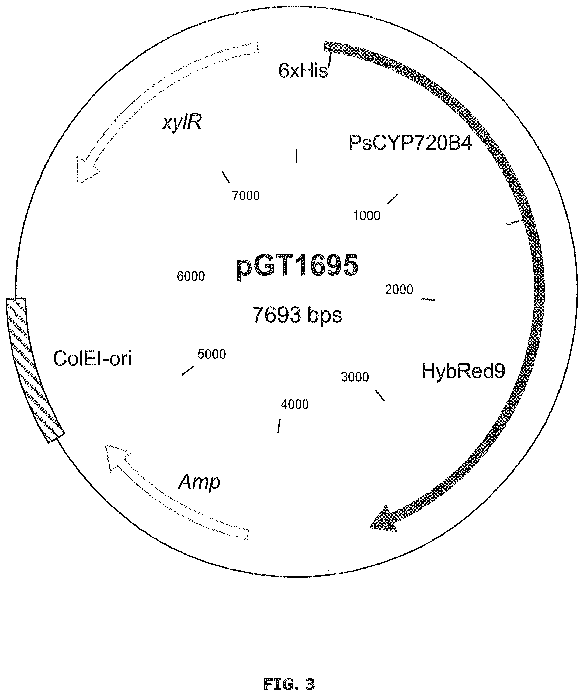

Figur 3: Plasmid enthaltend die Enzymkaskade zur oxidativen Umsetzung von Levopimaradien zu Levopimarsäure, mit 6xHis: HIS-Tag-Sequenz; PsCYP720B4: Cytochrome-P450-Monooxygenase-Domäne; HybRed9: Cytochrom-Reduktase-Domäne; Amp: Ampicillin-Resistenzgen; ColEI-ori: Regulator der Plasmidreplikation (origin of replication); xylR: Regulator des Xylosepromotors.

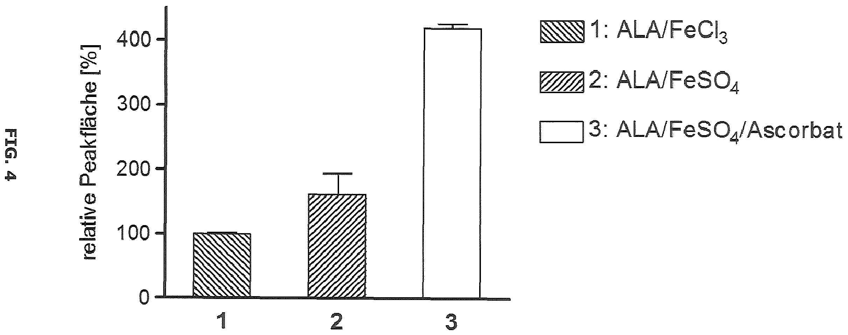

Figur 4: Medienzusatz Ascorbat bewirkt deutliche Steigerung der Produktausbeute. Eisenionen und Aminolävulinsäure sind Medienzusätze, die für eine hohe CYP-Aktivität benötigt werden. Die Verwendung von FeSO₄ (Fe²⁺) an Stelle von FeCl₃ (Fe³⁺) ist vorteilhaft (Ausbeutesteigerung: 60%). Zusatz des Antioxidans Ascorbat bewirkt eine weitere 2,6-fache Steigerung der Ausbeute an Harzsäure.

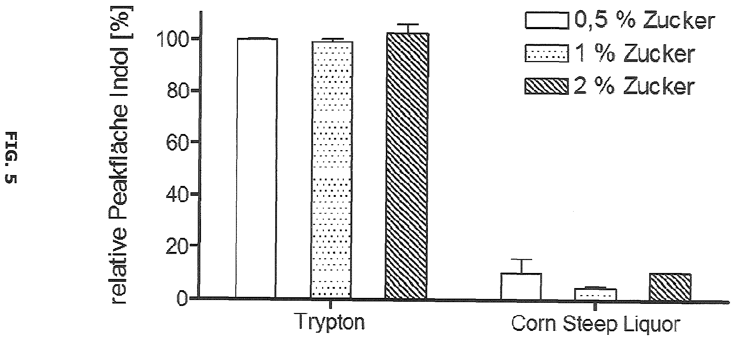

Figur 5: Minimierung des unerwünschten Stoffwechsel-Nebenprodukts Indol durch Verwendung einer geeigneten Stickstoffquelle. Unabhängig vom Gehalt der eingesetzten C-Quelle wird bei Ersatz der N-Quelle Trypton durch Cornsteep-liquor die Indolbildung um 90 bis 95% gesenkt. Die Indol-Quantifizierung erfolgte über HPLC.

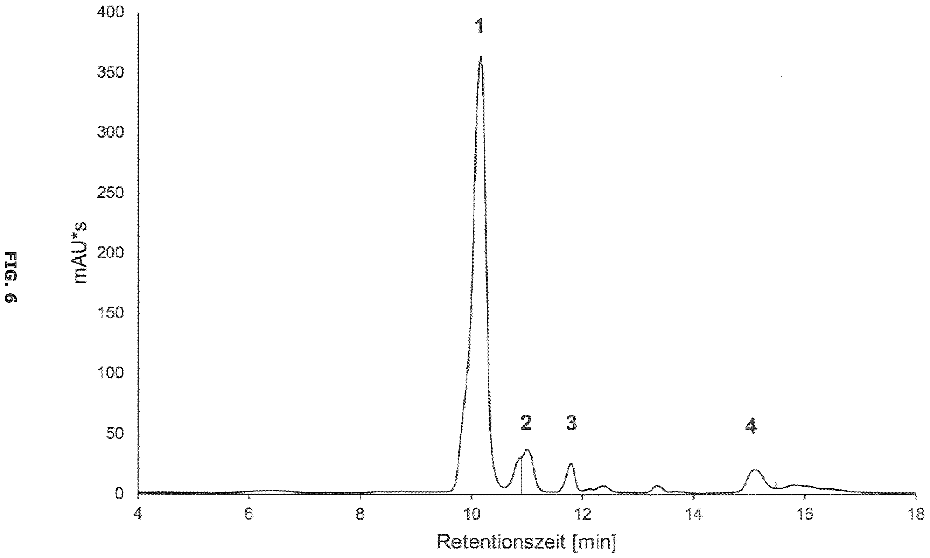

Figur 6: Typische HPLC-Analytik für erfindungsgemäß fermentativ hergestellte Levopimarsäure. Levopimarsäure wird durch sukzessive Oxidation von Levopimaradien über Levopimaradienol und Levopimaradienal generiert, wobei die Zwischenstufen zu geringen Anteilen im finalen Produkt enthalten sein können. Die 'Mengenanteile der oxidierten Produkte sind angegeben. 1: Levopimarsäure: 88,9 %, 2: Levopimardienal: 6,9 % 3: Levopimardienol: 4,2 %, 4: Levopimardien (Ausgangsverbindung)

### Detaillierte Beschreibung der Erfindung

Die vorliegende Erfindung betrifft den oben unter (1) beschriebenen, rekombinanten Mikroorganismus.
Unter einem "rekombinanten Mikroorganismus" ist im Zusammenhang mit der vorliegenden Erfindung ein gentechnisch veränderter bzw. modifizierter Mikroorganismus zu verstehen, der in seinem Genom heterologe Nukleotidsequenzen enthält, also Nukleotidsequenzen, die so natürlicherweise nicht in dem Mikroorganismus vorkommen. Hierzu zählen insbesondere Gene eines anderen Organismus (sogenannte Transgene), aber auch im Vergleich zur natürlichen Sequenz veränderte Nukleotidsequenzen und auch Nukleotidsequenzen, die natürlicherweise an einer anderen Stelle im Genom des Mikroorganismus vorhanden sind. Diese heterologen Nukleotidsequenzen können entweder auf einem oder mehreren Chromosomen des Mikroorganismus und/oder auf einem oder mehreren extrachromosomalen DNA-Elementen des Mikroorganismus vorliegen. Beispiele für extrachromosomale DNA-Elemente umfassen alle Arten von Vektoren, wie Plasmide, Cosmide, Bacterial Artificial Chromosomes (BACs), Yeast Artificial Chromosomes (YACs) oder virale Vektoren. Mikroorganismen im Sinne der vorliegenden Erfindung sind prokaryotische Mikroorganismen, insbesondere Bakterien, aber auch eukaryotische Mikroorganismen, wie Hefen oder andere Pilze. Ein bevorzugter prokaryotischer Mikroorganismus ist ein Bakterium, besonders bevorzugt ausgewählt aus der Gruppe bestehend aus fakultativ anaeroben Bakterien, insbesondere Proteobacteria, zum Beispiel der Gattung *Escherichia,* bevorzugt der Art *E. coli. E. coli* Stämme von besonderem Interesse im Zusammenhang mit dieser Erfindung sind *E. coli* BL21 und *E. coli* TOP10. Die gentechnische Veränderung der Mikroorganismen kann nach jeder der im Stand der Technik bekannten biotechnologischen Verfahren erfolgen, so etwa mittels Transformation, Transduktion oder Konjugation.

Der erfindungsgemäße rekombinante Mikroorganismus weist die Enzymaktivitäten wenigstens einer Geranylgeranyl-Pyrophosphat-Synthase (GGPPS), wenigstens einer Isopentenyl-Pyrophosphat-Isomerase (IPI), wenigstens einer Diterpensynthase (diTPS), wenigstens einer Cytochrom-P450-Monooxygenase (CYP), und wenigstens einer Cytochrom-P450-Reduktase (CPR) auf. Die entsprechenden Enzyme können dabei entweder natürlicherweise in dem Mikroorganismus vorkommen oder auch nicht, also entweder endogen oder heterolog sein, wobei aber wenigstens eines der Enzyme heterolog ist. Insbesondere sind auch Kombinationen von endogenen und heterologen Enzymen von der Erfindung mitumfasst.
Eine "Geranylgeranyl-Pyrophosphat-Synthase (GGPPS)" im Sinne der vorliegenden Erfindung ist ein Enzym, das die Synthese von Geranylgeranyl-Pyrophosphat (GGPP) aus Dimethylallylpyrophosphat (DMAPP) und Isopentenylpyrophospat (IPP) katalysiert. In einer Ausführungsform der vorliegenden Erfindung stammt die GGPPS aus Bakterien, bevorzugt aus Enterobakterien, besonders bevorzugt aus *Pantoea agglomerans.* Besonders bevorzugte Ausführungsformen der GGPPS sind solche, die eine Aminosäuresequenz gemäß SEQ ID NO:4 oder eine Aminosäuresequenz mit wenigstens 50 %, 60 %, 70 %, 80 %, 90 %, 95 % oder 99 % Identität über die gesamte Länge von SEQ ID NO:4 umfassen oder daraus bestehen. In einer weiteren Ausführungsform wird die GGPPS von einer Nukleotidsequenz umfassend oder bestehend aus SEQ ID NO:3 oder einer Nukleotidsequenz, die wenigstens 50 %, 60 %, 70 %, 80 %, 90 %, 95 % oder 99 % Identität über die gesamte Länge von SEQ ID NO:3 aufweist, codiert.
Die GGPP Synthese kann durch die Expression einer IPP-Isomerase (IPI), welche die Isomerisierung von IPP zu DMAPP katalysiert, unterstützt werden. In einer Ausführungsform der vorliegenden Erfindung stammt die IPI aus Bakterien, bevorzugt aus Enterobakterien, besonders bevorzugt aus *Pantoea agglomerans.* Besonders bevorzugte Ausführungsformen der IPI sind solche, die eine Aminosäuresequenz gemäß SEQ ID NO:6 oder eine Aminosäuresequenz mit wenigstens 60 %, 70 %, 80 %, 90 %, 95 % oder 99 % Identität über die gesamte Länge von SEQ ID NO:6 umfassen oder daraus bestehen. In einer weiteren Ausführungsform wird die IPI von einer Nukleotidsequenz umfassend oder bestehend aus SEQ ID NO:5 oder einer Nukleotidsequenz, die wenigstens 50 %, 60 %, 70 %, 80 %, 90 %, 95 % oder 99 % Identität über die gesamte Länge von SEQ ID NO:5 aufweist, codiert. In einer weiteren Ausführungsform der vorliegenden Erfindung stammt die IPI aus Pflanzen, bevorzugt aus Zingiberales, besonders bevorzugt aus *Curcuma wenyujin.* Besonders bevorzugte Ausführungsformen der IPI sind solche, die eine Aminosäuresequenz gemäß SEQ ID NO:14 oder eine Aminosäuresequenz mit wenigstens 80 %, 90 %, 95 % oder 99 % Identität über die gesamte Länge von SEQ ID NO:14 umfassen oder daraus bestehen. In einer weiteren Ausführungsform wird die IPI von einer Nukleotidsequenz umfassend oder bestehend aus SEQ ID NO:13 oder einer Nukleotidsequenz, die wenigstens 50 %, 60 %, 70 %, 80 %, 90 %, 95 % oder 99 % Identität über die gesamte Länge von SEQ ID NOs:13 aufweist, codiert.
Eine "Diterpensynthase (diTPS)" im Sinne der vorliegenden Erfindung ist ein Enyzm, das GGPP in das zyklische Zwischenprodukt Copalylpyrophosphat (CPP) umwandelt, das dann über mehrere Oxidationsschritte zum Diterpen-Endprodukt umgebaut wird (Lafever u.a., Arch. Biochem. Biophys. 313: 139-149 (1994)). Eine Ausführungsform der vorliegenden Erfindung umfasst bifunktionale diTPS (bidTPS), die GGPP in zwei Schritten zum Dien zyklisieren, wobei zunächst das Zwischenprodukt Copalyldiphosphat (CPP) gebildet wird und anschließend die Modifizierung zum Dien erfolgt. Alternativ können auch monofunktionale diTPS (mdiTPS), die nur die Umwandlung von CPP zum Dien katalysieren, eingesetzt werden und zwar in Kombination mit einem zusätzlichen Enzym, einer CPP Synthase (CPS). Die Wahl der diTPS ist maßgeblich für die zu synthetisierende(n) Harzsäure(n). So erfolgt die Synthese der Levopimarsäure unter Verwendung einer Levopimardiensynthase (LPS). In einer Ausführungsform der vorliegenden Erfindung wird Levopimarsäure unter Verwendung einer pflanzlichen LPS synthetisiert, bevorzugt einer LPS aus Ginkgo, besonders bevorzugt aus *Ginkgo biloba.* Besonders bevorzugte Ausführungsformen der LPS sind solche, die eine Aminosäuresequenz gemäß SEQ ID NO:2 oder eine Aminosäuresequenz mit wenigstens 50 %, 60 %, 70 %, 80 %, 90 %, 95 % oder 99 % Identität über die gesamte Länge von SEQ ID NO:2 umfassen oder daraus bestehen. In einer weiteren Ausführungsform wird die LPS von einer Nukleotidsequenz umfassend oder bestehend aus SEQ ID NO:1 oder einer Nukleotidsequenz, die wenigstens 50 %, 60 %, 70 %, 80 %, 90 %, 95 % oder 99 % Identität über die gesamte Länge von SEQ ID NO:1 aufweist, codiert.

Die Synthese der Abietinsäure erfolgt unter Verwendung einer Abietadiensynthase (AS). In einer Ausführungsform der vorliegenden Erfindung wird Abietinsäure unter Verwendung einer pflanzlichen AS synthetisiert, bevorzugt einer AS aus Tanne, besonders bevorzugt aus *Abies grandis.* Besonders bevorzugte Ausführungsformen der AS sind solche, die eine Aminosäuresequenz gemäß SEQ ID NO:16 oder eine Aminosäuresequenz mit wenigstens 50 %, 60 %, 70 %, 80 %, 90 %, 95 % oder 99 % Identität über die gesamte Länge von SEQ ID NO:16 umfassen oder daraus bestehen. In einer weiteren Ausführungsform wird die AS von einer Nukleotidsequenz umfassend oder bestehend aus SEQ ID NO:15 oder einer Nukleotidsequenz, die wenigstens 50 %, 60 %, 70 %, 80 %, 90 %, 95 % oder 99 % Identität über die gesamte Länge von SEQ ID NO:15 aufweist, codiert.
Die Synthese der Pimarsäure/Isopimarsäure erfolgt unter Verwendung einer Pimaradien/Isopimaradiensynthase (PIM). In einer Ausführungsform der vorliegenden Erfindung werden Pimarsäure/Isopimarsäure unter Verwendung einer pflanzlichen PIM synthetisiert, bevorzugt einer PIM aus Kiefer, besonders bevorzugt aus *Pinus contorta.* Besonders bevorzugte Ausführungsformen der PIM sind solche, die eine Aminosäuresequenz gemäß SEQ ID NO:18 oder eine Aminosäuresequenz mit wenigstens 50 %, 60 %, 70 %, 80 %, 90 %, 95 % oder 99 % Identität über die gesamte Länge von SEQ ID NO:18 umfassen oder daraus bestehen. In einer weiteren Ausführungsform wird die PIM von einer Nukleotidsequenz umfassend oder bestehend aus SEQ ID NO:17 oder einer Nukleotidsequenz, die wenigstens 50 %, 60 %, 70 %, 80 %, 90 %, 95 % oder 99 % Identität über die gesamte Länge von SEQ ID NOs:17 aufweist, codiert.
Eine "Cytochrom P450 Monooxygenase (CYP)" im Sinne der vorliegenden Erfindung ist ein Enzym, das die Oxidation von Diterpen-Dienen zu Harzsäuren katalysiert. Bevorzugt sind die CYP pflanzlichen Ursprungs, insbesondere solche aus Koniferen oder dikotylen Pflanzen wie *Medicago,* Tomate oder Soja. Besonders bevorzugte Ausführungsformen der CYP sind solche aus *Picea sitchensis* und/oder solche, die eine Aminosäuresequenz gemäß SEQ ID NO:8 oder eine Aminosäuresequenz mit wenigstens 70 %, 80 %, 90 %, 95 % oder 99 % Identität über die gesamte Länge von SEQ ID NO:8 umfassen oder daraus bestehen. In einer weiteren Ausführungsform wird die CYP von einer Nukleotidsequenz umfassend oder bestehend aus SEQ ID NO:7 oder einer Nukleotidsequenz, die wenigstens 50 %, 60 %, 70 %, 80 %, 90 %, 95 % oder 99 % Identität über die gesamte Länge von SEQ ID NO:7 aufweist, codiert.
Cytochrom-P450-Enzyme sind für die Übertragung der Elektronen auf einen Redoxpartner angewiesen. Dies kann zum Beispiel eine Cytochrom P450 Oxidoreduktase (CPR) sein. Im Sinne der vorliegenden Erfindung ist eine CPR eine Oxidoreduktase, die als Redoxpartner der CYP dient. Bevorzugt sind pflanzliche CPRs, etwa solche aus *Arabidopsis* und Ingwer. Bevorzugt sind weiterhin hybride Reduktasen, die durch Neukombination der Proteindomänen verschiedener Reduktasen gebildet werden. So stellt beispielsweise SEQ ID NO:10 eine hybride Reduktase, die Proteindomänen aus *Arabidopsis* und Ingwer enthält, dar. Besonders bevorzugte Ausführungsformen der CPR sind solche, die eine Aminosäuresequenz gemäß SEQ ID NO:10 oder eine Aminosäuresequenz mit wenigstens 50 %, 60 %, 70 %, 80 %, 90 %, 95 % oder 99 % Identität über die gesamte Länge von SEQ ID NO:10 umfassen oder daraus bestehen. In einer weiteren Ausführungsform wird die CPR von einer Nukleotidsequenz umfassend oder bestehend aus SEQ ID NO:9 oder einer Nukleotidsequenz, die wenigstens 80 %, 90 %, 95 % oder 99 % Identität über die gesamte Länge von SEQ ID NO:10 aufweist, codiert.
Die CPR kann entweder als eigenständiges Protein exprimiert werden oder als Fusionsprotein zusammen mit der CYP.

Nukleotidsequenzen, die für die vorgehend beschriebenen Enzyme codieren, können funktionell mit einem oder mehreren regulatorischen Elementen verknüpft sein. Derartige regulatorische Elemente umfassen alle im Stand der Technik bekannten regulatorischen Elemente, die die Expression des jeweiligen Enzyms beeinflussen können. Insbesondere zählen hierzu Promotoren, beispielsweise konstitutive und regulatorische Promotoren, Terminatoren, Ribosomenbindungsstellen und Enhancer.
Die Expression der vorgehend beschriebenen Enzyme kann weiterhin durch Codon-Optimierung der codierenden Nukleotidsequenzen, also durch Anpassung an die dem rekombinanten Mikroorganismus eigene codon-usage, beeinflusst werden. Somit sind insbesondere codon-optimierte Varianten der für die Enzyme codierenden Nukleotidsequenzen von der vorliegenden Erfindung mitumfasst. So stellt beispielsweise SEQ ID NO:1 eine hinsichtlich des codon-usage von *E. coli* optimierte Variante der Levopimardiensynthase aus *Ginkgo biloba* dar.

Die vorliegende Erfindung betrifft auch das oben unter (2) beschriebene Verfahren zur fermentativen *de novo* Synthese von Harzsäuregemischen definierter Zusammensetzung, umfassend die Kultivierung eines rekombinanten Mikroorganismus gemäß obigem Aspekt (1).
"*De novo*" Synthese bedeutet hierbei, dass die erfindungsgemäßen Endprodukte, also die Harzsäuren bzw. Harzsäuregemische, ausgehend von einfachsten Vorläufermolekülen, insbesondere Kohlenstoffverbindungen wie Zucker, gebildet werden.
"Fermentativ" meint weiterhin, dass es sich um eine enzymatische Umwandlung der Vorläufermoleküle zu den erfindungsgemäßen Endprodukten handelt.
Das erfindungsgemäße Verfahren umfasst die Kultivierung des erfindungsgemäßen rekombinanten Mikroorganismus. Hierbei bezeichnet "Kultivierung" jede Form der Mikroorganismenkultur, die die Synthese der erfindungsgemäßen Harzsäuren gewährleistet. Die Kultivierung erfolgt in einem geeigneten Nährmedium. Ein solches Nährmedium enthält eine Kohlenstoffquelle, bevorzugt wenigstens einen Zucker, der besonders bevorzugt ausgewählt ist aus der Gruppe umfassend Xylose, Glucose, Glycerin, Lactose, Galactose, Arabinose und Fructose, am meisten bevorzugt ist Xylose. Weiter kann das Nährmedium eine Stickstoffquelle enthalten, wobei es bevorzugt ist, dass es sich um eine Tryptophan-arme Stickstoffquelle, etwa Maisquellwasser (corn steep liquor) handelt. Weiter kann das Nährmedium Zusätze enthalten, die die Aktivität der exprimierten Enzyme beeinflussen können, beispielsweise Ascorbat oder Eisenverbindungen. Die Temperatur, bei der die Kultivierung erfolgt, richtet sich grundsätzlich nach dem zu kultivierenden Mikroorganismus. Weiter ist der Einfluss der Temperatur auf die jeweiligen Enzymaktivitäten zu berücksichtigen. In einer bevorzugten Ausführungsform der vorliegenden Erfindung wird *E. coli* bei einer Temperatur von 26 °C kultiviert.
Das erfindungsgemäße Verfahren dient der Herstellung von Harzsäuregemischen definierter Zusammensetzung. Grundsätzlich ist das Verfahren für die Herstellung einer Vielzahl verschiedener Harzsäuregemische mit unterschiedlicher, definierter Zusammensetzung, geeignet. So ist das Verfahren etwa geeignet, Gemische umfassend und/oder im Wesentlichen bestehend aus Levopimarsäure zu erzeugen. Bevorzugt ist die Herstellung von Levopimarsäure bzw. eines Gemisches, das im Wesentlichen aus Levopimarsäure besteht. Wie oben dargestellt, bestimmt die Wahl der eingesetzten Terpensynthase die herstellbaren Produkte. Die Produktion von Levopimarsäure bzw. eines Gemisches, das im Wesentlichen aus Levopimarsäure besteht, gelingt durch den Einsatz einer Levopimardiensynthase, bevorzugt einer Levopimardiensynthase aus Ginkgo. Unter einem Gemisch, das im Wesentlichen aus Levopimarsäure besteht, ist in einer Ausführungsform der vorliegenden Erfindung ein Gemisch zu verstehen, das wenigstens 80 %, bevorzugt wenigstens 82 %, besonders bevorzugt zwischen 80 % und 95 %, ganz besonders bevorzugt zwischen 82 % und 90 % Levopimarsäure enthält, wobei sich die vorstehenden Prozentangaben auf die oxidierten Levopimardienprodukte und auf eine Bestimmung mittels HPLC Analytik, wie unten beispielhaft dargestellt, beziehen.

Die Erfindung wird in dem nachfolgenden Beispiel näher erläutert:

### Beispiel

### Fermentative de novo Synthese von Levopimarsäure

### Verwendeter Bakterienstamm/Wirt

### E. coli BL21 (DE3) gold

Genotyp: F⁻ *ompT gal dcm Ion hsdS_{B}*(*r_{B}⁻m_{B}⁻*) λ(DE3 [*lacI lacUV5-T7p07 ind1 sam7 nin5*]) [*malB⁺*]_{K-12}(λ^{S})

### Eingesetzte Plasmide

### pGT1681

Das Plasmid pGT1681, wie in FIG. 2 dargestellt, enthält die Enzymkaskade zur Biosynthese der Grundstruktur Levopimaradien.

Die benötigten Enzymfunktionen umfassen:
- eine Geranylgeranyl-diphosphat-Synthase (crtE)
- eine Isopentenyl-Diphosphat-Isomerase (IPI)
- die Terpensynthase Levopimardiensynthase (LPS)
Basierend auf dem Plasmid pAC-BETAipi (Cunninhgam and Gantt, Photosynth. Res. 92:245-259 (2007)) wurde ein artifizielles Operon erzeugt, das diese drei Enzyme kodiert. Hierzu wurde das vorhandene crtE-Gen gegen ein alternatives Gen aus *P.agglomerans* ausgetauscht, das im Experiment eine höhere Produktausbeute bewirkte. Zudem wurden die Gene crtY, crtI und crtB entfernt und durch das Gen GbLPS ersetzt. Das GbLPS-Gen stammt aus der Pflanze *Ginkgo biloba* und kodiert für eine plastidär lokalisierte Levopimardiensynthase. Für die Verwendung im fermentativen System wurden die für das Chloroplasten-Lokalisationssignal kodierenden Sequenzen entfernt und nur die für das reife Protein kodierende Sequenz in das Plasmid pGT1681 eingesetzt. Zudem wurde die DNA-Sequenz von GbLPS hinsichtlich des Codon-usage für die Expression in *E. coli* optimiert.

Experimentell wurde gefunden, dass eine Variante der verkürzten GbLPS, die die Punktmutationen M534I und Y608F trägt, 10x aktiver als das Wildtyp-Enzym ist. Die Nukleotidsequenz der in pGT1681 enthaltenen GbLPS ist in SEQ ID NO:1 gezeigt.

In der Folge sind weitere Verbesserungen des erhaltenen Plasmids vorgenommen worden, die die Produktsynthese optimieren. So wurde die intergenische Region zwischen den Genen IPI und GbLPS angepasst. Außerdem wurden die *Shine-Dalgarno*-Sequenzen vor crtE und IPI optimiert. Der artifizielle Terminator aTerm5 wurde eingefügt. Die vollständige Nukleotidsequenz von pGT1681 ist in SEQ ID NO:12 zu finden.

### pGT1695

Das Plasmid pGT1695, wie in FIG. 3 dargestellt, enthält die Enzymkaskade zur oxidativen Umsetzung von Levopimaradien zu Levopimarsäure
Die benötigten Enzymfunktionen umfassen:
- eine Cytochrom-P450-Monooxygenase (CYP), bevorzugt PsCYP720B4 (SEQ ID NOs:7, 8)
- eine Cytochrom-Reduktase (CPR), bevorzugt HybRed9 (SEQ ID NOs: 9, 10) Dieser Expressionsvektor codiert für ein Fusionsprotein aus CYP und CPR, wobei das Fusionsgen unter der Kontrolle des Xylose-induzierbaren Promotors pXYL1 steht.

Als CYP-Komponente wurde das Gen für die Monooxygenase CYP720B4 aus *Picea sitchensis eingesetzt.* Die Gensequenz ist verkürzt, sodass ein N-terminal um 29 Aminosäuren verkürztes Protein kodiert wird (Deletion der Transmembranhelix am N-Terminus). Das verwendete Gen ist codonoptimiert für die Expression in *E.coli* und seine Nukleotidsequenz in SEQ ID NO:7 gezeigt.

Als CPR-Komponente für das Fusionsprotein wird das Gen HybRed9 (SEQ ID NO:9) verwendet, um die Produktausbeute zu optimieren.

Die vollständige Nukleotidsequenz von pGT1695 ist in SEQ ID NO:11 zu finden.

Die Enzymkaskaden der Plasmide pGT1681 und 1695 können alternativ auch in das Genom des Wirtsstammes integriert werden. Auf selektive Antibiotika kann in diesem Fall bei der fermentativen Produktion (s.u.) verzichtet werden.

### Kulturmedien und Kultivierung/Fermentation

Die Medien und Kulturbedingungen sind für die Harzsäureproduktion mit dem genannten Mikroorganismus optimiert worden. Die Effekte der Verbesserungen sind in der Folge dargestellt:
- Der Stamm BL21 synthetisiert 8x mehr Levopimaradien und 3x mehr Levopimarsäure als der ursprünglich verwendete Stamm *E.coli* TOP10.
- Anpassung der Kultur-Temperatur auf 26 °C bewirkt verbesserte Oxidaseaktivität.
- Zugabe der Hämvorstufe Aminolävulinsäure (ALA) und FeCl₃ zum Medium mit positiver Wirkung auf die Ausbeute an aktivem CYP-Enzym. Resultiert in höherer Oxidaseaktivität und besserer Endproduktbildung.
- Ersatz von FeCl₃ durch FeSO₄ bewirkt 60%-ig höhere Produktausbeute (siehe FIG. 4).
- Verwendung von Ascorbat als Medienzusatz bewirkt eine weitere 2,6-fache Steigerung der Produktausbeute (siehe FIG. 4).
- Verwendung von Corn Steep Liquor statt Trypton als Aminosäure/Stickstoffquelle bewirkt eine 90 %-ige Senkung der Indol-Nebenproduktion (siehe FIG. 5).

### Medien:

### dYT -Medium

1,6 g/l Stickstoffquelle*
1,0 g/l Hefeextrakt
0.5 g/l NaCl
(+ 15 g/l Agar bei festen Medien)
pH 7,0 → autoklaviert
*Stickstoffquelle können Trypton, Corn steep liquor (CSL), Beef-Extract oder Ammoniumsulfat bzw. Ammoniumchlorid sein. Die Ammoniumsalze oder CSL werden bevorzugt.

### Antibiotika

100 mg/l Ampicillin
25 mg/l Chloramphenicol

### Medienzusätze

| Endkonzentration im Medium | |
|---|---|
| 1x | Makro-Elemente* |
| 250 µM | Aminolävulinsäure |
| 25 µM | FeCl₃ oder FeSO₄ |
| 10 mM | Ascorbat |
| 30 g/l | Xylose |

| | |
|---|---|
| *10x-Makro-Elemente: 98 g/l K₂HPO₄ 3 g/l Ammonium iron (III) citrate (Sigma F5879-100G) 21 g/l Citric acid Monohydrate → sterilfiltriert | |

### Durchführung der Fermentation

Zur Kultivierung der Harzsäure-produzierenden Bakterienkulturen im Labormaßstab werden die Plasmide pGT1681 und pGT1695 in chemisch kompetente Zellen von *E. coli* BL21 mittels Hitzeschock transformiert wie es dem Fachmann bekannt ist. Zur Selektion positiver Transformanten wird die Kultur auf dYT-Medium (fest) mit Antibiotika (Amp + Cm) ausplattiert und die Platten über Nacht bei 30 °C inkubiert.

Für die fermentative Produktion der Harzsäuren im Schüttelkolben werden zunächst im Reagenzglas Vorkulturen angeimpft. Hierzu werden 3 ml dYT-Medium (flüssig) mit Antibiotika mit jeweils einer Kolonie beimpft und für ca. 4 Stunden bis zu einer Zelldichte von OD600≈0,6-1 bei 30 °C und 200 Umdrehungen/Minute (rpm), sodass ausreichender Sauerstofftransfer in das Nährmedium gewährleistet ist, angezogen. Für die Hauptkultur werden 15 ml auf 26 °C vorgewärmtes dYT-Medium (flüssig) mit Zusätzen und Antibiotika im 100 ml-Erlenmeyerkolben mit entsprechendem Volumen Vorkultur beimpft, sodass eine Zelldichte von OD600=0,033 vorliegt. Nach 20 h Kultivierung bei 26 °C und 200 Umdrehungen/Minute werden weitere 30 g/l Xylose zugegeben, die Kultur für weitere 20 Stunden bei 26 °C und 200 rpm inkubiert. Danach erfolgt die Abtrennung der Produkt-haltigen Biomasse vom Nährmedium mittels Zentrifugation (20 min, 5000 x g).

Produktion im größeren Volumen kann alternativ im Fermenter (Fed-batch Fermentation) erfolgen. Die benötigte Xylose wird hierbei über den Feed dem Fermenter zu geführt. Xylose dient zugleich als Kohlenstoffquelle und Induktor für die Expression des Oxidasesystems.

### Harzsäure-Extraktion zur Analytik

Material:
Saline: 0,9 % NaCl
Aceton (p.a.)
n-Hexan (p.a.)
Acetonitril (gradient grade)

Um Harzsäuren, Aldehyd, Alkohol und Olefine aus der Expressionskultur zu extrahieren wird diese in ein 50 ml-Zentrifugengefäß überführt und 5 Minuten bei 400 Umdrehungen/Minute abzentrifugiert. Der Überstand enthält keine signifikanten Mengen an Harzsäuren und wird daher verworfen. Das Zellpellet wird in 400 µl 0,9 % NaCl (Saline) resuspendiert und in ein 2 ml-Zentrifugengefäß überführt. Zum Zellaufschluss werden 400 µl Aceton (p.a.) zugegeben und gut gemischt bis das Zellpellet vollständig resuspendiert ist. Folgende Schritte werden insgesamt dreimal durchgeführt. Zur Extraktion werden 400 µl n-Hexan zugegeben, gut gemischt und 10 Minuten geschüttelt, anschließend 20-25 Sekunden bei 14.000 Umdrehungen/Minute abzentrifugiert. Der organische Lösungsmittelüberstand wird abpipettiert und in einem 1,5 ml Zentrifugengefäß gesammelt. Dieser enthält die Harzsäuren und ihre Vorstufen. Bei den Ausschüttel-Schritten wird darauf geachtet, dass das Zellpellet vollständig resuspendiert wird.

### Alle Arbeiten erfolgen bei Raumtemperatur

Nach mehreren Optimierungsschritten und Versuchen u.a. mit Ethylacetat und Acetonitril als Lösungsmitten, hat sich gezeigt, dass durch Kombination von Zellaufschluss mit Aceton und Extraktion mit n-Hexan die Harzsäuren und ihre Vorstufen am effizientesten extrahiert werden können. Die Arbeitszeit wurde deutlich verkürzt und die Extraktion der Produkte erfolgt quantitativ, sodass die verbleibenden Reststoffe keine signifikanten Produktmengen mehr enthalten.

### HPLC-Analyse

Vorbereitend zur Analyse der erhaltenen Harzsäureprodukte wird der erzeugte n-Hexan-Extrakt in einer Vakuumzentrifuge bei 2000 Umdrehungen/Minute und 40 °C unter Vakuum für 30 Minuten bis zur Trockne eingedampft. Der Rückstand wird in 200 µl Acetonitril (gradient grade) gelöst und für die HPLC-Analytik über Spartan®-Spritzenvorsatzfilter (0,2 µm Porengröße) vorgereinigt, um feinste Feststoffverunreinigungen zu entfernen.

Der Nachweis der Harzsäuren und ihrer Vorstufen erfolgt mittels HPLC. Die Trennung erfolgt über einen Gradienten von 20 % bis 100 % Laufmittel B in 10 Minuten mit Laufmittel A: 20 % Acetonitril in Wasser + 0,25 % Essigsäure und Laufmittel B: 100 % Acetonitril + 0,25 % Essigsäure bei einer Flussrate von 1 ml/Minute über eine Purospher®Star PR 18e 5 µm LiChroCART® 125-4 Säule. Die Detektion erfolgt für Levopimaradien-Derivate bei 270 nm, für Abietadien-/Pimaradien-Derivate bei 240 nm.

Die Ergebnisse zeigen, dass bei Durchführung des erfindungsgemäßen fermentativen Verfahrens mit dem Enzym Levopimardiensynthase (LPS) die Levopimarsäure das Hauptprodukt darstellt. Dessen Anteil an den oxidierten Levopimardienprodukten beträgt typischerweise 82-90 %. Daneben treten die unmittelbaren Vorstufen geringerer Oxidationsstufe (Aldehyd (-dienal) bzw. Alkohol(-dienol)) mit Anteilen von jeweils 5-9 % im Produktprofil auf. Ein typisches Chromatogramm ist in FIG. 6 dargestellt.

## Patentansprüche

1. Rekombinanter Mikroorganismus zur fermentativen *de novo* Synthese von Harzsäuregemischen definierter Zusammensetzung,
wobei der rekombinante Mikroorganismus die Enzymaktivitäten
(i) wenigstens einer Geranylgeranyl-Pyrophosphat-Synthase (GGPPS),
(ii) wenigstens einer Isopentenyl-Pyrophosphat-Isomerase (IPI),
(iii) wenigstens einer Diterpensynthase (diTPS), welche die Zusammensetzung bestimmt,
(iv) wenigstens einer Cytochrom-P450-Monooxygenase (CYP),
und
(v) wenigstens einer Cytochrom-P450-Reduktase (CPR) aufweist.

2. Rekombinanter Mikroorganismus nach Anspruch 1, **dadurch gekennzeichnet, dass** wenigstens eine der Enzymaktivitäten durch eine heterologe Nukleotidsequenz codiert wird.

3. Rekombinanter Mikroorganismus nach Anspruch 1 oder 2, **dadurch gekennzeichnet, dass**
(i) das Harzsäuregemisch im Wesentlichen aus Levopimarsäure besteht und die diTPS eine Levopimardiensynthase ist; oder
(ii) das Harzsäuregemisch im Wesentlichen aus Abietinsäure besteht und die diTPS eine Abietadiensynthase ist; oder
(iii) das Harzsäuregemisch im Wesentlichen aus Pimarsäure/Isopimarsäure besteht und die diTPS eine Pimaradien/Isopimaradiensynthase ist.

4. Rekombinanter Mikroorganismus nach Anspruch 3, **dadurch gekennzeichnet, dass**
(i) die diTPS eine Aminosäuresequenz mit wenigstens 50 % Identität über die gesamte Länge von SEQ ID NO:2 umfasst, bevorzugt SEQ ID NO:2 umfasst; oder
(ii) die diTPS eine Aminosäuresequenz mit wenigstens 50 % Identität über die gesamte Länge von SEQ ID NO:16 umfasst, bevorzugt SEQ ID NO:16 umfasst; oder
(iii) die diTPS eine Aminosäuresequenz mit wenigstens 50 % Identität über die gesamte Länge von SEQ ID NO:18 umfasst, bevorzugt SEQ ID NO:18 umfasst.

5. Rekombinanter Mikroorganismus nach Anspruch 3 oder 4, **dadurch gekennzeichnet, dass**
(i) die diTPS von einer Nukleotidsequenz gemäß SEQ ID NO:1 oder einer Nukleotidsequenz mit wenigstens 50 % Identität über die gesamte Länge von SEQ ID NO:1 codiert wird; oder
(ii) die diTPS von einer Nukleotidsequenz gemäß SEQ ID NO:15 oder einer Nukleotidsequenz mit wenigstens 50 % Identität über die gesamte Länge von SEQ ID NO:15 codiert wird; oder
(iii) die diTPS von einer Nukleotidsequenz gemäß SEQ ID NO:17 oder einer Nukleotidsequenz mit wenigstens 50 % Identität über die gesamte Länge von SEQ ID NO:17 codiert wird.

6. Rekombinanter Mikroorganismus nach einem der Ansprüche 1 bis 5, **dadurch gekennzeichnet, dass**
(i) die GGPPS aus Bakterien stammt, bevorzugt aus Enterobakterien, besonders bevorzugt aus *Pantoea agglomerans*; und/oder
(ii) die IPI aus Bakterien stammt, bevorzugt aus Enterobakterien, besonders bevorzugt aus *Pantoea agglomerans,* oder die IPI aus Pflanzen stammt, bevorzugt aus Zingiberales, besonders bevorzugt aus *Curcuma wenyujin*; und/oder
(iii) die CYP aus Pflanzen stammt, bevorzugt aus Koniferen, besonders bevorzugt aus *Picea sitchensis*; und/oder
(iv) die CPR aus Zingiberales oder Barassicales stammt, bevorzugt aus *Zingiber officinale* oder *Arabidopsis thaliana,* besonders bevorzugt eine hybride Reduktase ist.

7. Rekombinanter Mikroorganismus nach einem der Ansprüche 1 bis 6, **dadurch gekennzeichnet, dass**
(i) die GGPPS eine Aminosäuresequenz mit wenigstens 50 % Identität über die gesamte Länge von SEQ ID NO:4 umfasst, bevorzugt die Aminosäuresequenz von SEQ ID NO:4 umfasst; und/oder
(ii) die IPI eine Aminosäuresequenz mit wenigstens 60 % Identität über die gesamte Länge von SEQ ID NO:6 oder 80 % Identität zu SEQ ID NO:14 umfasst, bevorzugt die Aminosäuresequenz von SEQ ID NO:6 oder SEQ ID NO:14 umfasst; und/oder
(iii) die CYP eine Aminosäuresequenz mit wenigstens 70 % Identität über die gesamte Länge von SEQ ID NO:8 umfasst, bevorzugt die Aminosäuresequenz von SEQ ID NO:8 umfasst; und/oder
(iv) die CPR eine Aminosäuresequenz mit wenigstens 80 % Identität über die gesamte Länge von SEQ ID NO:10 umfasst, bevorzugt SEQ ID NO:10 umfasst.

8. Rekombinanter Mikroorganismus nach einem der Ansprüche 1 bis 7, **dadurch gekennzeichnet, dass**
(i) die GGPPS von einer Nukleotidsequenz gemäß SEQ ID NO:3 oder einer Nukleotidsequenz mit wenigstens 40 % Identität über die gesamte Länge von SEQ ID NO:3 codiert wird; und/oder
(ii) die IPI von einer Nukleotidsequenz gemäß SEQ ID NO:5 oder SEQ ID NO:13 oder einer Nukleotidsequenz mit wenigstens 40 % Identität über die gesamte Länge von SEQ ID NO:5 oder SEQ ID NO:13 codiert wird; und/oder
(iii) die CYP von einer Nukleotidsequenz gemäß SEQ ID NO:7 oder einer Nukleotidsequenz mit wenigstens 40 % Identität über die gesamte Länge von SEQ ID NO:7 codiert wird; und/oder
(iv) die CPR von einer Nukleotidsequenz gemäß SEQ ID NO:9 oder einer Nukleotidsequenz mit wenigstens 40 % Identität über die gesamte Länge von SEQ ID NO:9 codiert wird.

9. Rekombinanter Mikroorganismus nach einem der Ansprüche 1 bis 8, **dadurch gekennzeichnet, dass** wenigstens eines der Enzyme von einem extrachromosomalen DNA Element codiert wird, bevorzugt von einem Plasmid.

10. Rekombinanter Mikroorganismus nach einem der Ansprüche 2, 5 oder 8, **dadurch gekennzeichnet, dass** die Nukleotidsequenz funktionell mit wenigstens einem regulatorischen Element verknüpft ist, bevorzugt ausgewählt aus der Gruppe bestehend aus einem Promoter, einem Terminator, einem Enhancer, einer Ribosomenbindungsstelle.

11. Rekombinanter Mikroorganismus nach einem der Ansprüche 1 bis 10, der ein Bakterium, bevorzugt aus der Gattung *Escherichia,* besonders bevorzugt ein *E. coli* Stamm ist.

12. Verfahren zur fermentativen *de novo* Synthese von Harzsäuregemischen definierter Zusammensetzung, umfassend die Kultivierung eines rekombinanten Mikroorganismus nach einem der Ansprüche 1 bis 11.

13. Verfahren nach Anspruch 12, **dadurch gekennzeichnet, dass** die Kultivierung in einem Nährmedium erfolgt, das eine Kohlenstoffquelle, bevorzugt Zucker, besonders bevorzugt Xylose, Glucose, Glycerin, Lactose, Galactose, Arabinose oder Fructose, enthält.

14. Verfahren nach Anspruch 13, **dadurch gekennzeichnet, dass** das Nährmedium eine Stickstoffquelle, bevorzugt eine Tryptophan-arme Stickstoffquelle, besonders bevorzugt Maisquellwasser enthält.

15. Verwendung eines rekombinanten Mikroorganismus nach einem der Ansprüche 1 bis 11 zur fermentativen *de novo* Synthese von Harzsäuren sowie Harzsäuregemischen definierter Zusammensetzung.
